# EUROPEAN PATENT SPECIFICATION

(11) **EP 0 869 784 B1**
(45) Date of publication and mention of the grant of the patent: **31.08.2005**
(21) Application number: 96939542.5
(22) Date of filing: 15.11.1996
(51) Int. Cl.: A61K 31/365, A61P 35/00, A61P 3/04

(54) **INHIBITION OF FATTY ACID SYNTHASE AS A MEANS TO REDUCE ADIPOCYTE MASS**
HEMMUNG DER FETTSÄURESYNTHASE ALS MITTEL ZUR VERMINDERUNG DER ADIPOZYTENMENGE
INHIBITION DE SYNTHASE D'ACIDE GRAS DESTINEE A REDUIRE LA MASSE ADIPOCYTE

(30) Priority: 17.11.1995 US 6940 P
(43) Date of publication of application: 14.10.1998
(73) Proprietor: THE JOHNS HOPKINS UNIVERSITY, Baltimore, MD 21205-2109 (US)
(72) Inventor: KUHAJDA, Francis, P., Lutherville, MD 21209 (US); PASTERNACK, Gary, R., Baltimore, MD 21212 (US); TOWNSEND, Craig, A., Baltimore, MD 21212 (US); MANI, Neelakandha, S., Apartment C, Baltimore, MD 21209 (US)
(74) Representative: Dean, John Paul
(86) International application number: PCT/US1996/017678
(87) International publication number: WO 1997/018806

(56) References cited:
- WO-A-94/02108
- US-A- 4 028 397
- US-A- 4 218 443
- US-A- 5 032 611
- US-A- 5 246 960
- US-A- 5 447 954
- TRISCARI, J. ET AL: "Anti-obesity effect of a novel lipid synthesis inhibitor" INTERNATIONAL JOURNALOF OBESITY, vol. 8, no. suppl.1, 1984, pages 227-239, XP000961079
- TRISCARI, J. ET AL: "Antiobesity effects of a novel lipid synthesis inhibitor (RO 22-0654)" LIFE SCIENCES, vol. 34, no. 25, 1984, pages 2433-2442, XP000961103
- MCCUNE, S.A. ET AL: "Age-related decrease in sensitivity to glucagon and dibutyryl cyclic AMP inhibition of fatty acid synthesis in hepatocytes isolated from obese female Zucker rats" HORMONE AND METABOLIC RESEARCH, vol. 16, no. 2, 1984, pages 79-84, XP000961070
- PARK, B.K. ET AL: "Methylenolactocin, a novel antitumor antibiotic from Penicillium sp" THE JOURNAL OF ANTIBIOTICS, vol. 41, no. 6, 1984, pages 751-758, XP000971070
- GREENWOOD, M.R.C. ET AL: "Effect of (-)-hydroxycitrate on development of obesity in the Zucker obese rat" AMERICAN JOURNAL OF PHYSIOLOGY, vol. 240, no. 1, 1981, pages E72-E78, XP000961071
- WHITTINGTON, F.M. ET AL: "Effect of sodium 2-n-pentadecyl-benzimidazole-5-carboxylate (M & B 35347B), an inhibitor of acetyl-CoA carboxylase, on lipogenesis and fat deposition in obese hypergycaemic (ob/ob) an lean mice" INTERNATIONAL JOURNAL OF OBESITY, vol. 11, 1987, page 619-629 XP000961117
- HARRIS, R.B.S. ET AL: "In vitro evidence for an inhibitor of lipogenesis in serum from overfed obese rats" AMERICAN JOURNAL OF PHYSIOLOGY, vol. 257, no. 2 part 2, 1989, pages R326-R336, XP000961080

## Description

### Field of the Invention

This invention concerns the systemic administration of an inhibitor of fatty acid synthase (E.C. 2.3.1.85, FAS) by any suitable route to achieve weight loss and/or reduction of adipocyte mass without significant toxicity. Use of FAS inhibitors for this purpose is not restricted to humans or any species, but includes reduction of adipocyte mass in livestock and poultry. This invention also concerns the synthesis of a family of compounds (α-methylene-β-carboxy-γ-butyrolactones) which are fatty acid synthesis inhibitors, and the use of these compounds to achieve weight loss, to treat susceptible cancer cells, or in other applications characteristic of FAS inhibitors.

### Review of Related Art

Compounds and proteins which induce reduction of weight or adipocyte mass in mammals may act through the following mechanisms: [1] increased fat mobilization, such as human chorionic gonadotrophin (HCG) induced metabolism of fat stores (Bray, et al., "Nutritional Support of Medical Practice," Harper and Row, Philadelphia, 1983); [2] increased calorigenesis, such as that induced by thyroid hormone (Abraham et al., 1985, *Int. J. Obes.,* **9**:433-42); [3] decreased appetite, induced by anorexic agents such as felbamate and dexfenfluramine (McTavish et al., 1992, *Drugs,* **43**:713-33); [4] decreased lipogenesis (see Table 1 below); and [5] mutated or decreased blood levels of the *ob* gene product (Halaas et al., 1995, *Science,* **269**:543-546). In mice (Kannan et al., 1980, *Lipids,* **15**:993-8) and man, significant fatty acid synthesis occurs in liver (Triscari et al., 1985, *Metabolism,* **34**:580-7; Barakat et al., 1991, *Metabolism,* **40**:280-5) and adipose tissue (Goldrick et al., 1974, *Clin. Sci. Mol. Med,* **46**:469-79), and rates of fatty acid synthesis are higher in obese mice or humans (Angel et al., 1979, *Eur. J. Clin. Invest*., **9**:355-62; Belfiore et al., 1976, *Metabolism*, **25**:483-93). Hence, it is not surprising that a number of weight reducing agents reduce *de novo* fatty acid synthesis. Table 1 provides a list of agents known to both inhibit lipogenesis and induce weight loss.

**Table 1.**

| **Compounds Which Induce Weight Loss** | | |
|---|---|---|
| **COMPOUND** | **ENZYME TARGET and TYPE OF INHIBITION** | **REFERENCES and SPECIES STUDIED** |
| TPIA & CPIB | acetyl-CoA carboxylase, mixed competitive and (non-competitive) | Maragoudakis, 1969, *J. Biol. Chem,* **244**:5005-13 (rodent study) |
| Sodium 2-n-pentadecyl-benzimidazole-5-carboxylate | acetyl-CoA carboxylase, (competitive (?)) | Whittington et al., 1987, *Int. J. Obes.,* **11**:619-29 (rodent study) |
| Dehydroepiandosterone | glucose-6-phosphate dehydrogenase, (competitive) | Yen et al., 1981, *Lipids,* **12**:409-13 (rodent study) |
| (-)hydroxycitrate | ATP citrate lyase, (competitive) | Greenwood et al., 1981, *Am. J. Physiol.,* **240**:E72-8. (rodent study) |
| 30 kD protein | enzyme target unknown, inhibits FA synthesis | Harris, et al., 1989, *Am. J. Physiol*., **257**:R326-36 (rodent study) |
| *ob* gene product | protein produced by adipocytes which inhibits appetite | Halaas et al., 1995, *Sciences,* **269**:543-6 (rodent study) |

Interestingly, no inhibitors of fatty acid synthase (FAS) (E.C. 2.3.1.85) are mentioned in prior art as agents which produce weight loss.

### SUMMARY OF THE INVENTION

It is an object of this invention to provide a non-therapeutic method for inducing weight reduction focused on a new therapeutic target.

It is a further object of this invention to provide novel compounds that inhibit the activity of FAS; in particular, compounds with increased bioavailability and very low toxicity, compared to previously available FAS inhibitory compounds.

This invention relates to a method for inducing weight loss in an animal comprising administering to the animal a compound which reduces fatty acid synthase (FAS) activity in adipocytes or liver cells, the compound being other than a fat or a metabolizable product of a fat (i.e., the compound does not inhibit FAS through product inhibition). This invention also relates to a method for treating a condition responsive to reduction in adipose tissue mass in an animal comprising administering to the animal a composition which reduces fatty acid synthase (FAS) activity in adipocytes or liver cells, wherein the composition does not contain a fatty acid or fatty acid residue. The condition treated by this method may be obesity or non-insulin dependent diabetes mellitus. The composition may be administered in an amount sufficient to reduce adipocyte mass in the animal, or in an amount sufficient to reduce FAS activity in liver cells of the animal.

This invention also relates to a method for inducing weight loss in an animal comprising administering to the animal an inhibitor of fatty acid synthase (FAS), the FAS inhibitor preferably being administered in an amount sufficient to reduce fatty acid synthesis in adipose tissue or liver.

The present invention provides a pharmaceutical composition comprising a 5-substituted-2-oxo-3-methylene-4-furancarboxylic acid, wherein the substituent is selected from:
(a) a saturated linear alkyl group of 3-18 carbons;
(b) a saturated branched alkyl group of 3-18 carbons;
(c) an unsaturated linear or branched alkyl group of 3-18 carbons;
(d) 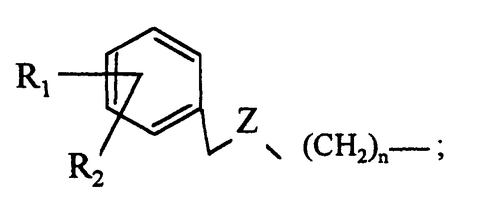
(e) 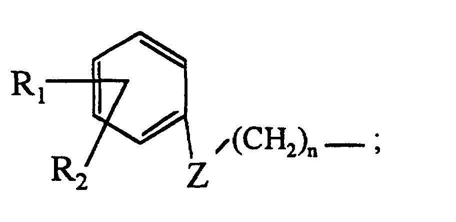
(f) 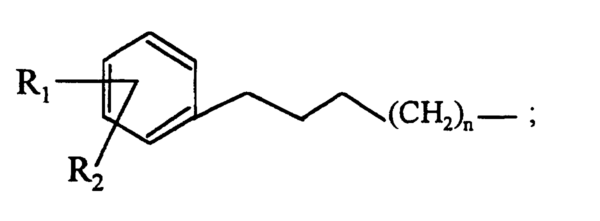
(g) 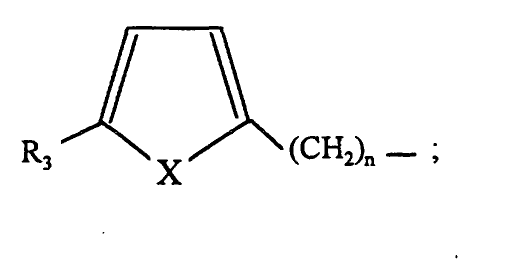
(h) 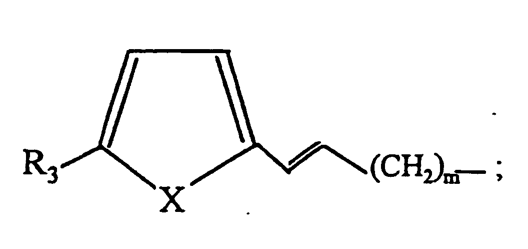
(i) 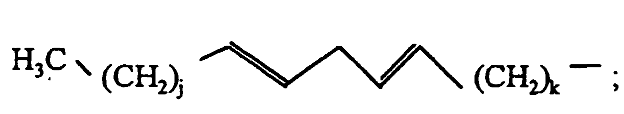 and
(j) 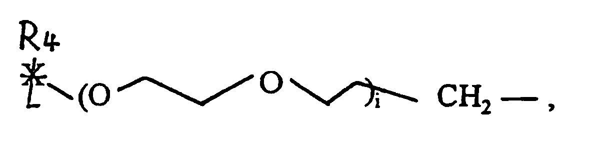
wherein R₁ and R₂ each are H, CH₃, C₂H₅, C₃H₇, C₄H₉, CF₃, OCH₃, F, Cl, or Br, R₃ is H, CH₃, C₂H₅, C₃H₇, C₄H₉, COOH, COOCH₃, COOC₂H₅, COOC₃H₇, or COOC₄H₉; R₄ is H, CH₃, C₂H₅, C₃H₇, C₄H₉; X is NH, S or O; Z is CH₂, O, NH or S; i is 1 to 5; j is 0 to 10; k is 1 to 10; m is 1-13; and n is 1 to 15, and R1 and R2 may be the same or different, wherein the 5-substituted-2-oxo-3-methylene-4-furancarboxylic acid is not methylenolactin. In a preferred embodiment, the substituent is C₄H₉, C₃H₇, C₆H₁₃, C₇H₁₅, C₈H₁₇, C₉H₁₉, C₁₀H₂₁, C₁₂H₂₅, C₁₄H₂₉, C₁₅H₃₁, C₁₆H₃₃, C₁₇H₃₅, C₁₈H₃₇, 3E-4-(3-fluorophenyl) or 3E,6E-octadienyl. In a particularly preferred embodiment, the substituent is n-octyl.

In another embodiment, this invention provides a method for inducing weight loss in an animal comprising administering to the animal a pharmaceutical composition containing the 5-substituted 2-oxo-3-methylene-4-furancarboxylic acid as defined in claim 5 below.

In yet another embodiment, this invention provides a method of inhibiting growth of tumor cells in an animal, said cells expressing at least one enzyme of the fatty acid biosynthetic pathway, comprising administering a pharmaceutical composition containing the 5-substituted 2-oxo-3-methylene-4-furancarboxylic acid as defined in claim 8, to the tumor cells. Typically the tumor cells are cells which express fatty acid synthase (FAS), and preferably, the composition is administered in an amount specifically cytotoxic to said tumor cells.

No literature prior to the present studies has identified FAS as a target enzyme for weight reduction. However, we have noted weight loss in nude mice treated with cerulenin, a non-competitive inhibitor of FAS. Sustained reduction of adipocyte mass in humans without toxicity would significantly impact disease prevention world-wide. Aside from psychological and self-esteem improvement, weight loss via reduction of adipocyte mass may [1] ameliorate hyperglycemia associated with non-insulin-dependent diabetes mellitus thereby reducing diabetic complications such as arterial disease, blindness, cataracts, etc., [2] reduce hypertension, [3] reduce risk of coronary artery vascular disease and stroke, and [4] reduce the risk of other complications of massive obesity such as osteoarthritis, surgical complications, etc. There is also potential use in livestock and poultry to reduce the saturated fat content of meat products. Therefore FAS inhibitors are disclosed herein as novel agents for weight reduction.

A family of compounds (γ-substituted-α-methylene-β-carboxy-γ-butyrolactones) whose synthesis was based on the cerulenin motif is shown herein to inhibit fatty acid synthesis, inhibit growth in certain susceptible tumor cells, and induce weight loss. The α-methylene-β-carboxy-γ-butyrolactones have several advantages over the natural product cerulenin for therapeutic applications: [1] they do not contain the highly reactive epoxide group of cerulenin, [2] they are stable and soluble in aqueous solution, [3] they can be produced by a two step synthetic reaction and thus easily produced in gram quantities, and [4] they are easily tritiated to high specific activity for biochemical and pharmacologic analyses. Thus, this family of compounds is disclosed herein for these therapeutic applications.

### BRIEF DESCRIPTION OF THE FIGURES

Figure 1 shows induction of weight loss in nude mice by cerulenin.
Figure 2 shows cerulenin-induced weight loss in nude mice inoculated with OVCAR-3 cells.
Figure 3 shows C-75 inhibition of acetate incorporation into acylglycerides by HL60 cells.
Figure 4 shows C-75-induced weight loss in nude mice inoculated with OVCAR-3 cells and monitored for 40 days.
Figure 5 shows rescue of HL60 cells from C-75 inhibition by exogenous fatty acid.
Figure 6 shows C-75-induced weight loss in nude mice inoculated with OVCAR-3 cells and monitored for 19 days.
Figure 7 shows Kaplan-Meier Survival Curves for nude mice inoculated with OVCAR-3 cells with or without C-75 treatment.
Figure 8 shows maximum weight loss vs. survival for individual mice.
Figure 9 shows Kaplan-Meier Survival Curves for nude mice inoculated with OVCAR-3 cells with or without a single C-75 treatment.

### DETAILED DISCLOSURE OF THE INVENTION

This invention relates to [1] the use of inhibitors of fatty acid synthase (E.C. 2.3.1.85, FAS), such as the inhibitor cerlenin, as a means to reduce body weight; [2] the synthesis of a family of compounds (α-methylene-β-carboxy-γ-butyrolactones) which are fatty acid synthesis inhibitors; [3] the use of α-methylene-β-carboxy-γ-butyrolactone fatty acid synthesis inhibitors as a means to treat tumor cells expressing FAS; [4] the use of α-methylene-β-carboxy-γ-butyrolactone fatty acid synthesis inhibitors as a means to reduce body weight; and [5] the use of any fatty acid synthase inhibitors to systematically reduce adipocyte mass (adipocyte cell number or size) as a means to reduce body weight.

### FAS Inhibitors

FAS inhibitors contemplated by this invention are compounds that directly reduce the activity of FAS in animal cells without any significant (direct) effect on other cellular activities, at least at comparable concentrations. A wide variety of compounds have been shown to inhibit fatty acid synthase (FAS), and selection of a suitable FAS inhibitor for use in this invention is within the skill of the ordinary worker in this art. Compounds which inhibit FAS can be identified by testing the ability of a compound to inhibit fatty acid synthase activity using purified enzyme. Fatty acid synthase activity can be measured spectrophotometrically based on the oxidation of NADPH, or radioactively by measuring the incorporation of radiolabeled acetyl- or malonyl-CoA. (Dils, et al, Methods Enzymol, 35:74-83). FAS inhibitors are exemplified in U.S. patent application 08/188,425 and International patent publication WO 94/02108, both of which are incorporated herein by reference.

Suitable FAS inhibitors may be identified by a simple test exemplified in Example 7, and in U.S. patent application 08/188,425. Generally, this test uses a tumor cell line in which an FAS inhibitor, typically cerulenin, is cytotoxic. Such cell lines include SKBR-3, ZR-75-1, and preferably HL60. Suitable FAS inhibitors will inhibit growth of such cell lines, but the cells are rescued by exogenous supply of the product of the FAS enzyme (fatty acid). When cell growth is measured in the presence and absence of exogenous fatty acid (e.g., palmitate or oleate), inhibition by specific FAS inhibitors is relieved by the fatty acid.

Alternatively, suitable FAS inhibitors can be characterized by a high therapeutic index. Inhibitors can be characterized by the concentration required to inhibit fatty acid synthesis in cell culture by 50% (IC₅₀ or ID₅₀). FAS inhibitors with high therapeutic index will inhibit fatty acid synthesis at a lower concentration (as measured by IC₅₀) than the IC₅₀ for inhibition of cell growth in the presence of exogenous fatty acid. Inhibitors whose effects on these two cellular activities show greater differences are more preferred. Preferred inhibitors of fatty acid synthesis will have IC₅₀ for fatty acid synthetic activity that is at least 1 log lower, more preferably at least 2 logs lower, and even more preferably at least 3 logs lower than the inhibitor's IC₅₀ determined for cell growth.

### Therapy with FAS Inhibitors

Fatty acid synthesis inhibitors, especially FAS inhibitors, will block the body's ability to convert carbohydrates to fat. On a fat restricted diet, this will lead to a reduction of storage fat. In addition, it will lead to decreased intracellular fat storage and a reduction in adipocyte mass. This may be expected to have the primary and/or secondary effects listed in Table 2. Fatty acid synthesis inhibition will lead to reduction in hepatic fat, and this in turn can lead to reduction in the rate or incidence of cirrhosis in alcoholics (see, e.g., French, 1989, *Clinical Biochemistry,* **22**:41-9; Clements, et al., 1995, *Am. J. Respir. Crit. Care Med.,* **151**: 780-784, incorporated herein by reference). Increased insulin responsiveness is a direct consequence of decreased adipocyte mass. Reduced adipocyte mass will reduce the risk of arterial vascular disease, stroke, etc. Thus, the method of this invention is particularly applicable to overweight individuals, diabetics, and alcoholics. The method is generally useful as part of a program to treat obesity and complications thereof.

### Table 2. Effects of decreased intracellular fat storage and reduction in adipocyte mass

- Weight loss without muscle loss
- Reduction in hepatic fat
- Increased insulin responsiveness (especially in Type II diabetes mellitus)
- Decreased blood pressure
- Decreased arterial vascular disease
- Decreased susceptibility to liver injury associated with fatty change, including endotoxin mediated liver injury

The method of the present invention for inducing weight loss is applicable to animals, including vertebrates, especially mammals. Animals particularly contemplated include food animals such as poultry, swine, cattle, sheep, and other animals where reduction in fat accumulation without reduction in muscle mass may be desirable for veterinary health or economic reasons. Similarly, FAS inhibitors may be administered according to the method of this invention to dogs, cats, horses and other animals for veterinary health reasons, particularly reasons analogous to the reasons given herein for medical therapeutic use of this invention. Dosing protocols for the FAS inhibitors according to this method may be adapted to various animals from the medical procedures and the *in vitro* and *in vivo* data provided herein, in view of standard veterinary pharmacological principles. Generally, this method will not be applied to lactating animals.

Treatment according to this invention involves administering a compound according to this invention (an FAS inhibitor and/or an α-methylene-β-carboxy-γ-butyrolactone) to the subject of treatment. The pharmaceutical compositions containing any of the compounds of this invention may be administered by parenteral (subcutaneously, intramuscularly, intravenously, intraperitoneally, intrapleurally, intravesicularly or intrathecally), topical, oral, rectal, or nasal route, as necessitated by choice of drug and disease.

Therapeutic compounds according to this invention are preferably formulated in pharmaceutical compositions containing the compound and a pharmaceutically acceptable carrier. The concentrations of the active agent in pharmaceutically acceptable carriers will depend on solubilities. The dose used in a particular formulation or application will be determined by the requirements of the particular type of disease and the constraints imposed by the characteristics and capacities of the carrier materials. The pharmaceutical composition may contain other components so long as the other components do not reduce the effectiveness of the compound according to this invention so much that the therapy is negated. Pharmaceutically acceptable carriers are well known, and one skilled in the pharmaceutical art can easily select carriers suitable for particular routes of administration (see, e.g., Remington's Pharmaceutical Sciences, Mack Publishing Co., Easton, PA, 1985). Dose and duration of therapy will depend on a variety of factors, including the therapeutic index of the drugs, disease type, patient age, patient weight, and tolerance of toxicity. Dose will generally be chosen to achieve serum concentrations from about 1 ng to about 100 µg/ml, typically 0.1 µg/ml to 10 µg/ml. Preferably, initial dose levels will be selected based on their ability to achieve ambient concentrations shown to be effective in *in-vitro* models, such as that used to determine therapeutic index, and *in-vivo* models and in clinical trials, up to maximum tolerated levels. Standard clinical procedure prefers that chemotherapy be tailored to the individual patient and the systemic concentration of the chemotherapeutic agent be monitored regularly. The dose of a particular drug and duration of therapy for a particular patient can be determined by the skilled clinician using standard pharmacological approaches in view of the above factors. The response to treatment may be monitored by analysis of blood or body fluid levels of the compound according to this invention, measurement of activity if the compound or its levels in relevant tissues or monitoring disease state in the patient. The skilled clinician will adjust the dose and duration of therapy based on the response to treatment revealed by these measurements.

The compositions described above may be combined or used together or in coordination with another therapeutic substance. The inhibitor of fatty acid synthesis, or the synergistic combination of inhibitors, will of course be administered at a level (based on dose and duration of therapy) below the level that would kill the animal being treated. Preferably, administration will be at a level that will not irreversibly injure vital organs, or will not lead to a permanent reduction in liver function, kidney function, cardiopulmonary function, gastrointestinal function, genitourinary function, integumentary function, musculoskeletal function, or neurologic function. On the other hand, administration of inhibitors at a level that kills some cells which will subsequently be regenerated (e.g., endometrial cells) is not necessarily excluded.

### Suppression of FAS Biosynthesis

While the FAS inhibitors discussed herein are typically small molecule compounds that directly inhibit the enzyme, it will be readily apparent to the skilled clinician that specific prevention of FAS biosynthesis is an equivalent procedure which will accomplish the same desired result. Therefore, this invention also contemplates inhibition of FAS biosynthesis as a method for treating obesity, etc. This may be accomplished by selectively degrading mRNA encoding FAS or otherwise interfering with its transcription and/or translation. This may be accomplished, for instance, by introduction of a ribozyme specific for FAS mRNA (see, e.g., Czubayko, et al., 1994, *J. Biol. Chem.,* **269**:21358-21363, incorporated herein by reference, for ribozyme methodology). This may alternatively be accomplished by antisense RNA complementary to the nucleic acid sequence of FAS. The sequence of human FAS is disclosed in U.S. patent application 08/188,426, incorporated herein by reference. Typically, antisense therapy involves an expression vector containing at least a portion of the sequence encoding human FAS operably linked to a promoter such that it will be expressed in antisense orientation. As a result, RNA which is complementary to and capable of binding or hybridizing to FAS mRNA will be produced. Upon binding to mRNA for FAS, translation of that mRNA is prevented, and consequently FAS is not produced. Production and use of antisense expression vectors is described in more detail in U.S. Patent 5,107,065 and U.S. Patent 5,190,931, both of which are incorporated herein by reference.

The expression vector material is generally produced by culture of recombinant or transfected cells and formulated in a pharmacologically acceptable solution or suspension, which is usually a physiologically-compatible aqueous solution, or in coated tablets, tablets, capsules, suppositories, inhalation aerosols, or ampules, as described in the art, for example in U.S. Patent 4,446,128, incorporated herein by reference. The vector-containing composition is administered to a mammal in an amount sufficient to transfect a substantial portion of the target cells of the mammal. Administration may be any suitable route, including oral, rectal, intranasal or by intravesicular (e.g. bladder) instillation or injection where injection may be, for example, transdermal, subcutaneous, intramuscular or intravenous. Preferably, the expression vector is administered to the mammal so that the tumor cells of the mammal are preferentially transfected. Determination of the amount to be administered will involve consideration of infectivity of the vector, transfection efficiency *in vitro,* immune response of the patient, etc. A typical initial dose for administration would be 10-1000 micrograms when administered intravenously, intramuscularly, subcutaneously, intravesicularly, or in inhalation aerosol, 100 to 1000 micrograms by mouth, or 10⁵ to 10¹⁰ plaque forming units of a recombinant vector, although this amount may be adjusted by a clinician doing the administration as commonly occurs in the administration of other pharmacological agents. A single administration may usually be sufficient to produce a therapeutic effect, but multiple administrations may be necessary to assure continued response over a substantial period of time.

Further description of suitable methods of formulation and administration according to this invention may be found in U.S. Patents 4,592,002 and 4,920,209, incorporated herein by reference.

### Synthesis of α-methylene-β-carboxy-γ-butyrolactones

While experiments with cerulenin demonstrate that a fatty acid synthesis inhibitor induces both weight and adipocyte mass reduction *in vivo,* the bioavailability of cerulenin is limited, and this in turn may limit its use as a drug in humans. Rational design and synthesis of novel compositions of matter has lead to fatty acid synthesis inhibitors which have increased potency and stability *in vivo.* The design of inhibitors/inactivators of FAS is predicated upon: [1] an understanding of the mechanism of fatty acid biosynethesis, in particular that of the critical ketosynthase or "condensing enzyme" domain of this polyfunctional enzyme (Wakil, S.J., 1989, *Biochemistry,* **28**:4523-4530; Funabashi et al., 1983, *Tetrahedron,* **24**:2673-2676) and, [2] the well-studied inactivation of FAS by the natural product cerulenin (Funabashi et al., 1989, *J. Biochem.,* **105**:751-755).

In fatty acid biosynthesis the characteristic two-carbon chain extension is carried out by FAS. Malonate, bound as its thioester to the pantetheine arm of acyl carrier protein (ACP), enters the active site where the elongating fatty acid chain is bound to a highly reactive cysteine residue of the enzyme. The malonyl ACP is decarboxylated to give a reactive enolate anion, which attacks the cysteine-bound acyl group. The transiently generated tetrahedral oxyanion rapidly decomposes to regenerate the free enzyme cysteine thiolate and the homologated β-ketoacyl intermediate bound to ACP. The chemical complexity of these steps, that is (a) the role and mechanism of decarboxylation, (b) the generation of an oxyanion intermediate and (c) the presence of a highly reactive cysteine thiol(ate) in the catalytic cycle, make these steps in the biosynthesis especially attractive for drug design.

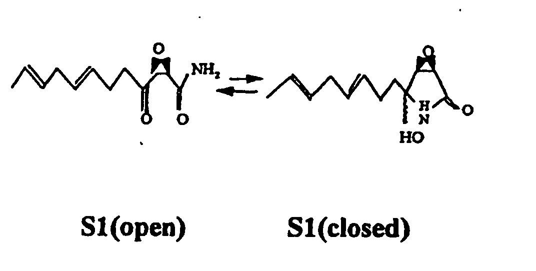

Nature chose to target the ketosynthase step of FAS in the natural antibiotic cerulenin (S1). Cerulenin exists in solution as a equilibrium between an open (S1-open) and closed form (S1-closed). Cerulenin is a potent inactivator of FAS and is known to covalently bind to the reactive cysteine residue of the ketosynthase domain. (Funabashi et al., 1989; D'Angnolo et al., 1973, *Biochim. Biophys. Acta*, **326**:155-166; Siggaard-Andersen et al., 1991, *Proc. Natl. Acad Sci. USA*, **88**:4114-4118).

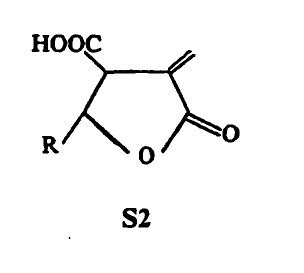

Simple molecular modeling exercises and structure-based searches based on the mechanism outlined above and in the key chemical elements noted above have yielded a small number of lead compounds, some known to possess FAS inhibitory properties specifically or antimicrobial/antitumor activities more generally. Among these was a group of α-methylene lactones of the general structure S2: protolichesteric acid (S2, R = C₁₃H₂₇) (Berdy, J., 1982, "Anonymous Handbook of Antibiotic Compounds", *CRC Press,* pp. 39-53), *allo*-perusaric acid (S2, R = C₁₃H₂₆ COCH₃) (Huneck et al., 1986, *Phytochem*., **25**:543-549), nephromopsinic acid (S2, R = C₁₁H₂₃) (Berdy, 1982) and methylenolactocin (S2, R =C₅H₁₁) (Park et al., 1988, *Antibiotics,* **41**:751-758). The last of these compounds, for example, has been isolated from a *Penicillium* species and found to possess antibacterial and antitumor activity ((Park et al., 1988), but the mechanism(s) of action is/are not understood. A series of derivatives of S2 containing side chains (R) of various lengths has been synthesized by the present inventors in recognition of the carboxylic acid and the suitably-placed electrophilic α-methylene lactone as potential inactivators of the ketosynthase of FAS.

A considerable body of synthetic information exists to prepare compounds of this structural type (Murta et al., 1993, *J. Org. Chem*., **58**:7537-7541; Azevedo et al., 1992, *J. Org. Chem*., **57**:4567-4569). Prominent among these was an impressively efficient route to the specific class represented by S2 reported by Carlson and Oyler (Carlson et aL, 1976, *J. Org. Chem*., **41**:4065-4069). In this synthesis the dianion of 4-methoxybenzyl itaconate (S3) can be condensed with aldehydes (S4) of various chain description (R) to give in *one* step, after rapid exposure to strong acid, the desired α-methylene lactone carboxylic acid S2. Both the *trans-* and cis-isomers are generated slightly favoring the former. These diastereomers may be separated by flash silica gel chromatography using ethyl acetate:hexanes:acetic acid (30:70:1) as eluent and individually crystallized from boiling hexanes.

A series of derivatives of S2 has been prepared containing saturated alkyl, unsaturated alkyl and aryl-containing side chains (R)( e.g., -C₆ H¹³-C₇H₁₅ -C₈H₁₇, -C₉H₁₉, -C₁₁H₂₃ and -C₁₃H₂₇). Based on their *in vitro* biological activity against HL60 cells, human breast cancer cell lines, and normal fibroblasts, the S2 compound with R = -C₈H₁₇ was chosen as a representative compound for further study. This compound, designated C-75 herein, coincidentally has an alkyl side chain approximately the length of cerulenin. C-75 has been prepared in crystalline form. For biochemical studies, radiolabeled S2 is readily accessible from the tritiated aldehyde S4 (H* = ³H), which is in turn easily available by reduction with sodium borohydride(³H) and reoxidation to the aldehyde. A substantial kinetic isotope effect in the latter reaction is well known and leads to high retention of the heavy isotope as demonstrated in earlier work from this laboratory (Townsead et al., 1986, *J. Chem. Soc. Chem. Commun*., 638-639). If higher specific radioactivity is required, more elaborate catalytic tritiation of a carbon-carbon double bond could be carried out to give the aldehyde at very high specific activity.

The preparation of these compounds and survey of various structural types may be amenable to combinatorial methods by use of the resin supports **S5** and **S6**. 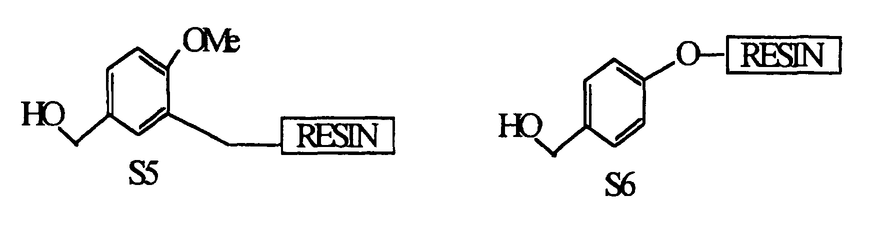 Esterification with itaconic anhydride would yield the ester corresponding to **S3** ready for dianion formation and reaction with aldehydes **S4.** Unreacted aldehyde can be washed from the resin and the product obtained by acidification to cause cyclization to the lactone **S2** and regeneration of the resins **S5** and **S6.** Structural types of aldehydes represented above and by others should be assayed as described below to exclude inactive forms.

Alpha-methylene-β-carboxy-γ-butyrolactones, including tetrahydro-3-methylene-2-oxo-5-n-octyl-4-furancarboxylic acid (C-75), are easily and inexpensively synthesized and can be radiolabeled to high specific activity for *in vivo* pharmaokinetic analysis. Like cerulenin, C-75, and similar α-methylene-β-carboxy-γ-butyrolactones, will effectively inhibit the growth of tumor cells *in vivo,* particularly those such as OVCAR-3 cells which express high levels of FAS, and these compounds can be used in treating cancers containing such cells (see U.S. patent application No. 08/188,425, incorporated herein by reference).

### EXAMPLES

In order to facilitate a more complete understanding of the invention, a number of Examples are provided below. However, the scope of the invention is not limited to specific embodiments disclosed in these Examples, which are for purposes of illustration only.

### Example 1. Synthesis of α-methylene-β-carboxy-γ-butyrolactones.

This example illustrates the use of the 4-methoxybenzyl itaconate dianion S3 for preparation of several derivatives of type S2 and S2¹.

### Example 1A. Preparation ofp-methoxybenzyl itaconate.

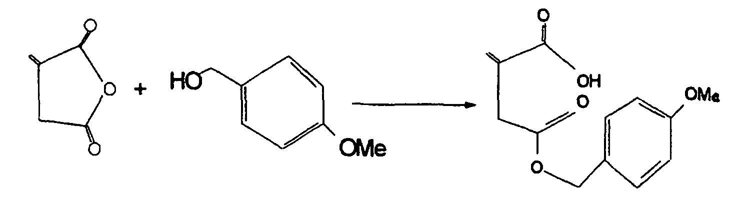

A mixture of itaconic anhydride (19 g, 0.169 mole) and p-methoxybenzyl alcohol (50 mL) was stirred in a 250 mL round-bottomed flask at 55-60 °C for 40 h. After cooling to room temperature, the reaction mixture was diluted with 150 mL of diethyl ether and the solution was poured into saturated aqueous sodium bicarbonate (200 mL). The layers were separated and the aqueous layer was acidified to pH 3 with conc. HC1. The precipitate formed was collected by filtration and dried in *vacuuo*. Recrystallization from ethyl acetate-hexane furnished 38 g of the desired ester as a white crystalline solid (90%); Mp 87.5 °C (lit 86.8-87.2 °C, Carson, et al., 1976); IR (film) 3000-3400, 2935, 1720, 1681, 1634, 1612, 1515 cm⁻¹; ¹H NMR δ (CDCl₃) 3.3 (s, 2H), 3.7 (s, 3H), 5.0 (s, 2H), 5.7 (d, 1H, *J* = 0.8 Hzz), 6.43 (s, 1H), 6.80 (d, 2H, *J* = 8.8 Hz), 7.24 (d, 2H, *J* = 8.4 Hz).

### Example 1B. Preparation of α-methylene lactone derivatives―typical procedure

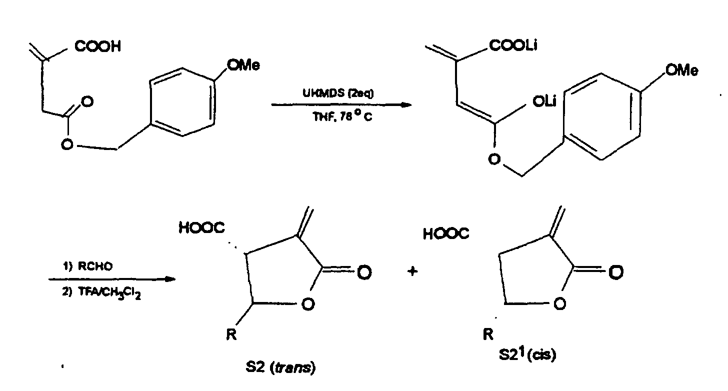

Lithiumhexamethyldisilyl amide (40 ML, 1M, 40 mmol) was added to a solution of 20 mmol of the itaconate half ester in 200 ML of anydrous THF cooled at -78 °C. After stirring for 1h, 20 mmol of the aldehyde (RCHO dissolved in 10 mL of anhydrous THF) was introduced to the reaction mixture through a cooled (-78 °C) cannula. After the addition, the solution was stirred at -78 °C for 3-4 h, and then the reaction was quenched with 20 mL of 6N H₂SO₄ and immediately extracted into either (250 mL). The ethereal solution was dried over anhydrous MgSO₄. The solution was filtered and evaporated under reduced pressure yielding a gummy solid. This crude product was taken up in CH₂Cl₂ (100-125 mL) and treated with 1.5 mL of trifluoroacetic acid. The mixture was stirred at room temperature for 10-12 h. Aqueous NaHCO₃ (100 mL) was then added to the reaction and the layers were separated. The bicarbonate layer was acidified to pH 1 with conc. HCl and extracted with ether (2 x 100 mL). The organic layers were dried over anhydrous MgSO₄, filtered and evaporated under reduced pressure to yield the α-methylene lactones as a mixture of *cis-* and *trans*-diastereoisomers as a white solid in overall yields ranging from 58-67%.

The predominant trans-isomer (55-60%) was separated by flash column chromatography using silica gel (ethyl acetate:hexanes:acetic acid 30:70:1). The products were further purified by recrystallization from boiling heaxanes.

### Example 1C. trans-Tetrahydro-3-methylene-2-oxo-5-n-octyl-4-furancarboxylic acid (C-75)

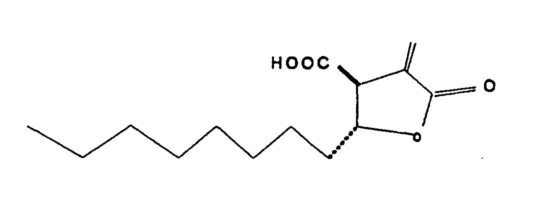

mp 76-77 °C (hexane); IR (film) 3000-3400, 2924, 2852, 1743, 1717, 1660, 1621, 1460 cm⁻¹; ¹H NMR (CDCl₃) δ 0.84 (1, 3H, *J* = 6.8 Hz), 1.2-1.8 (m, 14H), 3.59 (dt, 1H, *J -* 2.8, 5.6, 12.8 Hz), 4.77 (q, 1H, *J =* 6, 12.8 Hz), 6.0 (d, 1H, *J* = 2.8 Hz), 6.4 (d, 1H, *J*= 3.2 Hz); ¹³C NMR (CDCl₃) δ 14.0, 22.6, 24.7, 29.1, 29.14, 31.7, 35.1, 49.4, 78.7, 125.9, 132.2, 168.1, 174.5; exact mass: calculated = 254.1518, found = 254.1514.

***cis*****-isomer:** mp 74-75.5 °C (hexane); IR (film) 3000-3400, 2922, 2855, 1748, 1713, 1663 cm⁻¹; ¹H NMR (CDCl₃) δ 0.7 (b, 3H), 1.2-1.8 (b, 14H), 4.0 (dt, 1H, *J* = 2, 7.6 Hz), 4.6 (q, 1H, *J =* 5.6, 7.6 Hz), 5.9 (d, *J* = 2.4 Hz), 6.4 (d, 1H, *J* = 2.0 Hz); ¹³C NMR (CDCl₃) δ 14.0, 22.6, 25.5, 29.1, 29.14, 29.3, 31.3, 31.7, 48.8, 78.0, 125.7, 133.1, 168.8, 174.4.

### Example 1D. trans-Tetrahydro-3-methylene-2-oxo-5-n-hexyl-4-furancarboxylic acid

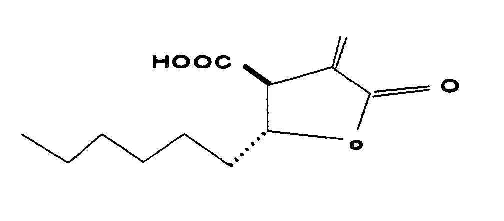

IR (neat) 3000-3400, 2953, 2852, 1743, 1717 1660, 1256, 1460 cm⁻¹; ¹H NMR (CDCl₃) δ 0.8 (bt, 3H) 1.20-1.81 (m, 10H), 3.59 (dt, 1H, J= 2.8, 5.6 Hz), 4.76 (q, 1H, *J* = 6.0, 2.8 Hz), 5.91 (d, 1H, *J =* 2.8 Hz), 6.42 (d, 1H, *J* = 2.8 Hz); ¹³C NMR (CDCl₃) δ 13.9, 22.4, 24.6, 28.7, 31.5, 35.6, 49.4, 78.9, 126.0, 132.2, 168.4, 174.5.

**Cis isomer:** IR (neat) 3000-3400, 2922, 2855, 1748, 1713, 1663, 1466 cm⁻¹; ¹H NMR (CDCl₃) δ 0.7 (b, 3H), 1.2-1.8 (b, 10H), 4.0 (bd, 1H, *J*= 7.6 Hz), 4.6 (q, 1H, *J* = 5.6, 7.6 Hz), 5.9 (d, *J* = 2.4 Hz), 6.4 (d, 1H, *J* = 2.4 Hz).

### Example 1E. trans-Tetrahydro-3-methylene-2-oxo-5-n-heptyl-4-furancarboxylic acid

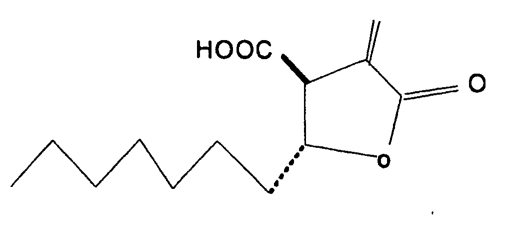

IR (neat) 3000-3400, 2960, 2840, 1747, 1654, 1460 cm⁻¹; ¹H NMR (CDCl₃) δ 0.7 (b, 3H), 1.3-1.8 (m, 12H), 3.6 (dt, 1H, *J* = 2.8, 5.6 Hz), 4.79 (q, 1H, *J* = 5.6, 12.8 Hz), 6.0 (d, 1H, *J* = 2.4 Hz), 6.45 (d, 1H, *J* = 2.8 Hz).

### Example 1F. trans-Tetrahydro-3-methylene-2-oxo-5-n-nonyl-4-furancarboxylic acid

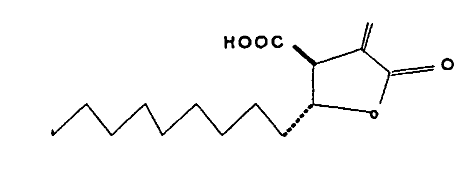

IR (film) 3000-3400, 2924, 2852, 1743, 1717, 1660, 1460 cm⁻¹; ¹H NMR (CDCl₃) δ 0.84 (t, 3H, *J=* 6.8 Hz), 1.2-1.8 (m, 16H), 3.59 (dt, 1H, *J=* 2.8, 5.6 Hz), 4.77 (q, 1H, *J =* 6, 12.8 Hz), 6.0 (d, 1H, *J* = 2.8 Hz), 6.4 (d, 1H, *J* = 3.2 Hz); ¹³C NMR (CDCl₃) δ 14.1, 22.6, 24.7, 29.1, 29.2, 29.23, 29.3, 29.4, 31.8, 35.7, 49.4, 78.8, 126.0, 132.2, 168.1, 174.7.

### Example 1G. trans-Tetrahydro-3-methylene-2-oxo-5-n-undecyl-4-furancarboxylic acid

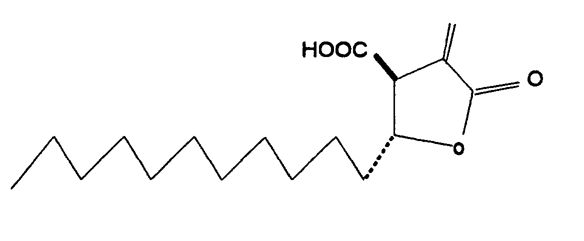

IR (film) 3000-3400, 2924, 2852, 1745, 1717, 1660, 1460 cm⁻¹; ¹H NMR (CDCl₃) δ 0.84 (t, 3H, *J* = 6.8 Hz), 1.2-1.8 (m, 20H), 3.6 (dt, 1H, *J* = 2.8 Hz, 5.6 Hz), 4.77 (q, 1H, *J* = 2.8, 5.6, 12.8 Hz), 6.0 (d, 1H, *J* = 2.8 Hz), 6.4 (d, 1H, *J* = 2.8 Hz); ¹³C NMR (CDCl₃) δ 14.0, 22.6, 24.7, 29.1, 29.19, 29.3, 29.4, 29.47, 29.5, 31.8, 35.7 49.4, 78.8, 126.0, 132.2, 168.3, 174.8.

### Example 1H. trans-Tetrahydro-3-methylene-2-oxo-5-n-tridecyl-4-furancarboxylic acid

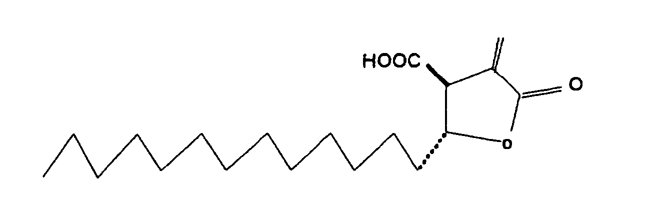

IR (film) 3000-3400, 2919, 2850, 1749, 1719, 1661, 1467 cm⁻¹; ¹H NMR (CDCl₃) δ 0.84 (t, 3H, *J =* 6.8 Hz), 1.2-1.8 (m, 20H), 3.60 (dt, 1H, *J =* 2.8 Hz, 5.6 Hz), 4.77 (q, 1H, *J* = 5.6, 12.8 Hz), 6.10 (d, 1H, *J =* 2.8 Hz), 6.4 (d, 1H, *J* = 2.8 Hz); ¹³C NMR (CDCl₃) δ 14.0, 22.6, 24.7, 29.1, 29.2, 29.3, 29.38, 29.4, 29.48, 29.5, 29.58, 29.62, 31.8, 35.7, 49.4, 78.8, 125.9, 132.3, 168.3, 174.8.

### Example 1I. trans-Tetrahydro-3-methylene-2-oxo-5-(3E, 6E-octadieny)-4-furancarboxylic acid

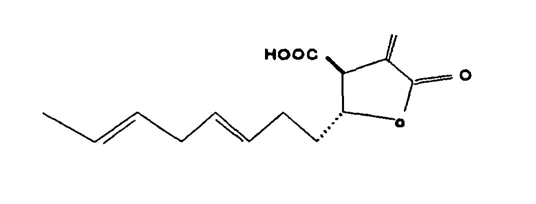

IR (neat) 3000-3400, 2924, 1743, 1717, 1660, 1610, 1465 cm-¹; ¹H NMR (CDCl₃) d 1.61 (d, 3H), 1.70-2.25 (m, 4H), 2.65 (m, 2H), 3.61 (dt, 1H, *J* = 2.8, 5.6 Hz), 4.80 (q, 1H, *J* = 5.5, 12.6 Hz), 5.2-5.4 (m, 4H), 6.05 (d, 1H, *J* = 2.8 Hz), 6.38 (d, 1H, *J* o= 2.8 Hz).

### Example 1J. trans-Tetrahydro-3-methylene-2-oxo-5-(3E-4-(3-fluorophenyl))but-3-enyl-4-furancarboxylic acid

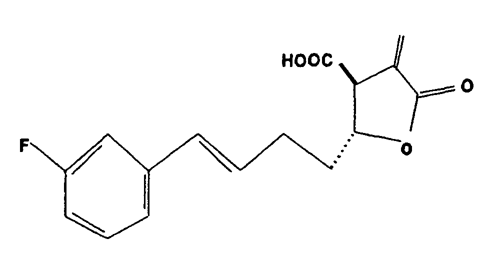

IR (neat) 3000-3400, 2917, 2877, 1749, 1717, 1660, 1500 cm⁻¹; ¹H NMR (CDCl₃) δ 1.80-2.65 (m, 2H), 3.60 (dt, 1H, *J* = 2.8, 5.6 Hz), 4.80 (q, 1H, *J* = 5.5, 12.6 Hz), 6.04-6.42 (m, 4H), 6.75-7.38 (m, 4H).

### Example 2. Cerulenin induces weight loss in athymic nude mice.

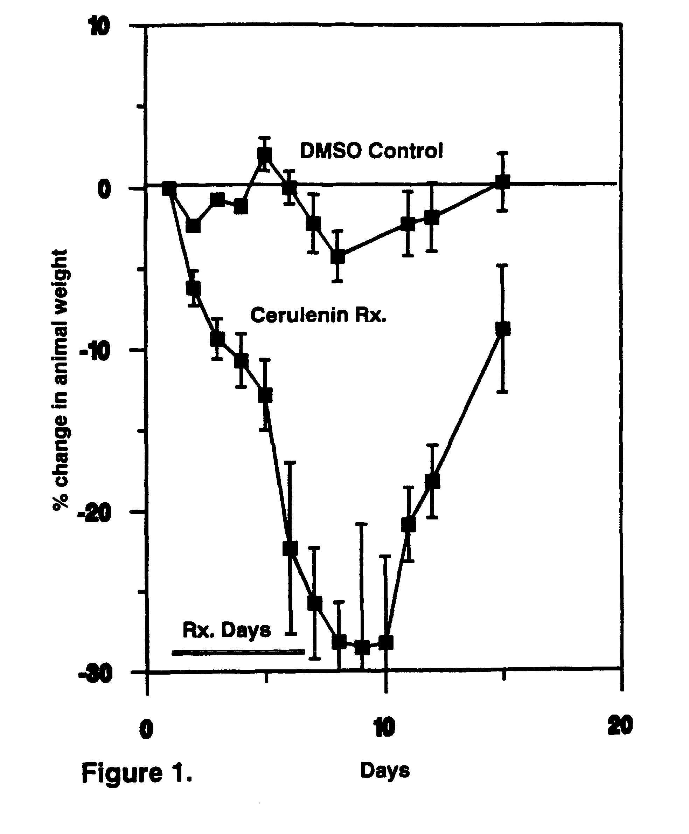

Athymic mice received 60 mg/kg cerulenin i.p. in 25% DMSO/saline or vehicle alone each day for 1 week. The animal weights were recorded every two days up to day 15. Average animal weight for both groups of mice is plotted against treatment day in Figure 1. Error bars represent standard error of the mean. Intraperitoneal administration of cerulenin for seven days to nude mice resulted in an average 28% loss of weight by day eight, with mice returning to 96% of their original weight by day 14 (Figure 1). In addition there was a 28% reduction of feces mass in the cerulenin-treated group consistent with deceased food intake. Water intake was not appreciable altered. Observation of the mice showed normal activity even during the period of maximal weight loss.

Necropsy of treated animals from a parallel experiment showed markedly decreased subcutaneous fat and abdominal fat deposits, and reduced liver volume consistent with reduction in hepatic fat accumulation. Microscopically, the liver was unremarkable without necrosis or inflammation but cerulenin-treated animals had an absence of the fat droplets which were seen in controls. In addition, white fat deposits around abdominal organs were significantly reduced, but brown fat deposits remained. While the white fat deposits were reduced, no decrease in muscle mass was detected histologically.

### Example 3. Cerulenin induces weight loss in nude mice OVCAR-3 xenografts.

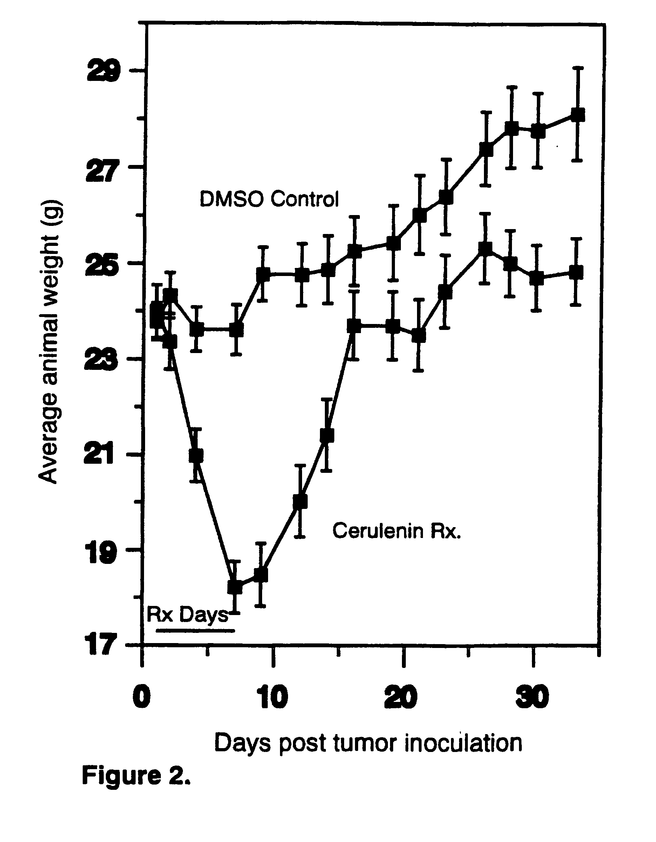

Cerulenin was administered as described in Example 2 above to mice which received 10⁷ OVCAR-3 human ovarian cancer cells. Athymic mice received 0.1 ml of packed NIH-OVCAR-3 cells (approximately 10⁵ cells) intraperitoneally on treatment day 0. Beginning on day 1, treated mice received 80 mg/kg cerulenin i.p. in 25% DMSO/saline for 1 week, while untreated mice received vehicle alone. As shown in Figure 2, cerulenin-treated mice experienced a mean weight loss of 25% of original body mass during the treatment period. Weight loss constitutes the major toxicity observed during fatty acid synthesis inhibition therapy with cerlenin; the mice regained inception body mass within 10 days following treatment. Importantly, urinalysis failed to demonstrate any diabetogenic effect of cerulenin.

### Example 4. Cerulenin inhibits fatty acid synthesis in vivo.

Fatty acid synthesis was measured using metabolic labeling *in vivo* with [U-³H]-acetate as described in Pizer, et al., 1996, *Cancer Res.,* **56**:1189-1193. OVCAR-3 ascites tumor fatty acid synthesis was reduced by 43%, after intraperitoneal administration of cerulenin performed as in Example 3, demonstrating that cerulenin inhibits fatty acid synthesis *in vivo.* No augmentation of inhibition was achieved with high dose cerulenin [6mg/mouse] over the standard dose [2mg/mouse], indicating that drug activity was probably maximal. There was no effect on mean hepatic fatty acid synthesis after cerulenin due to the short half life (<15 min.) of the drug in ascites..

In parallel experiments using mice without tumor, inhibition of fatty acid synthesis in the range of 50% was observed in liver. Thus, the reversible cerulenin-induced weight loss may be the result of fatty acid synthesis inhibition as well as decreased appetite in the mice.

### Example 5. C-75 inhibition of fatty acid synthesis.

Incubation of C-75 (50 µg/ml) with a hypotonic lysate (20 mM K₂PO₄ pH 6.6 at 25 °C) of OVCAR-3 ascites cells at 37 ° C for 180 min results in a 3-fold reduction of FAS activity compared to control lysate (5.6 nM NADPH oxidized/min by control; 1.8 with C-75) as measured by monitoring the malonyl-CoA dependent oxidation of NADPH at 340 nm U.V. Incubation at 4° abolishes the inhibitory effect of C-75 while not affecting the activity of the controls. While relatively high concentrations of C-75 were required in the lysate to achieve inhibition of FAS; in a living cell, the effect of C-75 may be more efficient, requiring lower concentrations of compound.

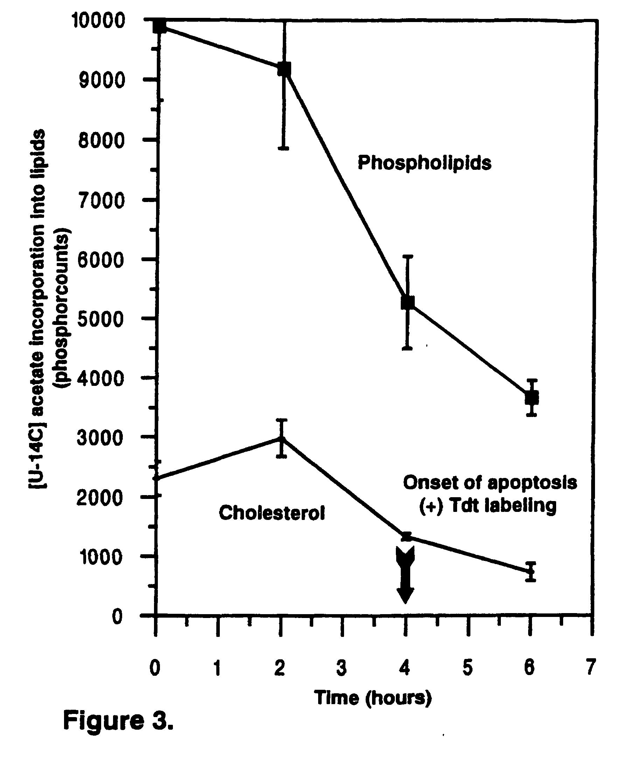

*In vitro* studies in HL60 human promyelocytic leukemia cells demonstrated that C-75 inhibits fatty add synthesis. HL60 cells were labeled with [U¹⁴C]-acetate in the presence of 10 µg/ml C-75; cells were incubated in 5% CO₂ at 37°C. Results are plotted in Figure 3; each time point was performed in triplicate, error bars are the standard deviation. Figure 3 shows a time dependent reduction of label incorporation into phospholipids, representing nearly a three-fold reduction in fatty acid synthesis. Pilot *in vivo* metabolic labeling studies have demonstrated that C-75 reduces [U-³H] acetate incorporation into fatty acids in mouse liver by 30%.

### Example 6. C-75 induces weight loss in vivo.

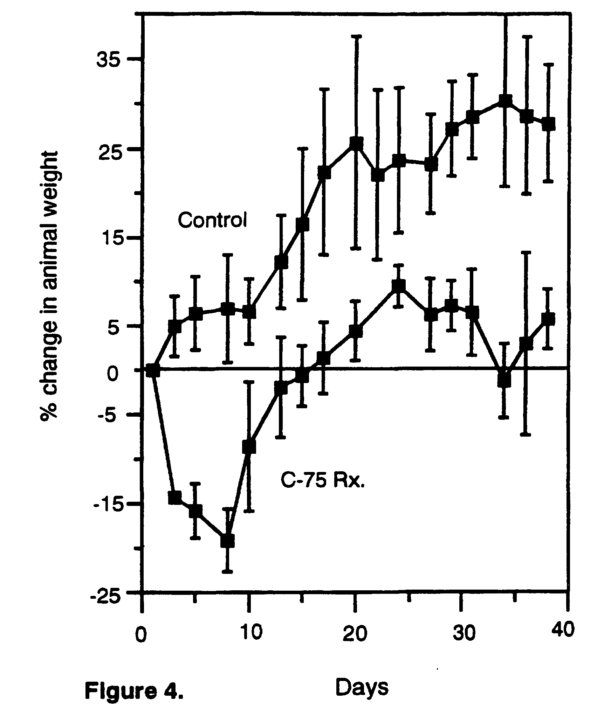

C-75 was tested *in vivo* in a pilot study against the OVCAR-3 xenograft model of Example 4. Ten mice were inoculated intraperitoneally with 10⁷ OVCAR-3 cells. The mice were treated on days 1, 3, and 5 with either DMSO or 60 mg/kg C-75. Results are plotted in Figure 4. C-75 treated mice experienced a 20% reduction in weight loss by day eight. Animals regained their original weight by day 15, increased their weight to nearly 110% of original body weight by day 24, and achieved original body weight by day 34.

### Example 7. C-75 inhibition of fatty acid synthesis by HL60 cells.

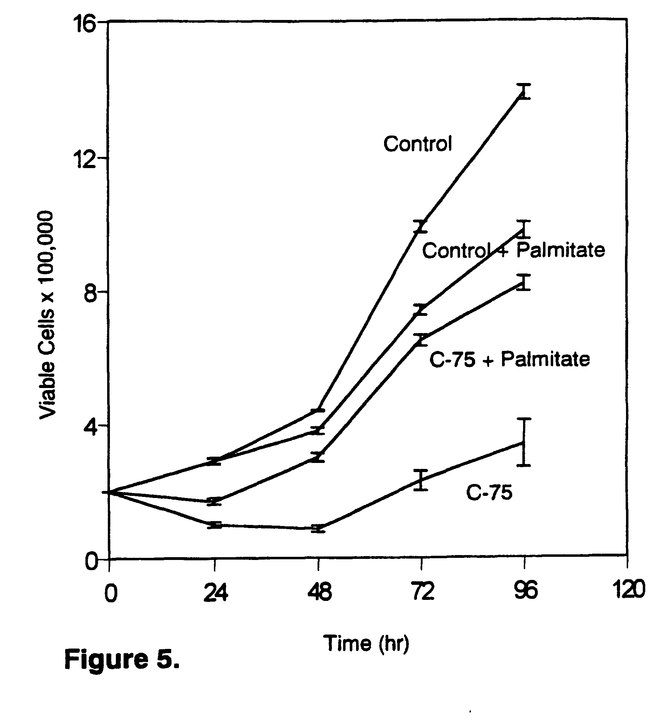

*In vitro* studies in HL60 human promyelocytic leukemia cells demonstrate that C-75 inhibition of fatty add synthesis can be reversed by exogenous fatty acid addition. 200,000 HL60 cells were plated in 24- well plates in 1 ml fatty acid free medium with or without palmitate (80 micromolar, complexed to fatty acid free albumin 0.2:1). Cells were treated with 2.5 ug/ml of C-75. Cells were incubated in 5% CO₂ at 37°C and viable cells were counted with trypan blue exclusion at 24, 28, 72, and 96 hours. Results are plotted in Figure 5; each time point was performed in triplicate, error bars are the standard deviation. Figure 5 shows that palmitate rescues HL60 cells from C-75. Note that palmitate alone causes some suppression of HL60 proliferation.

### Example 8. C-75 induces weight loss in vivo.

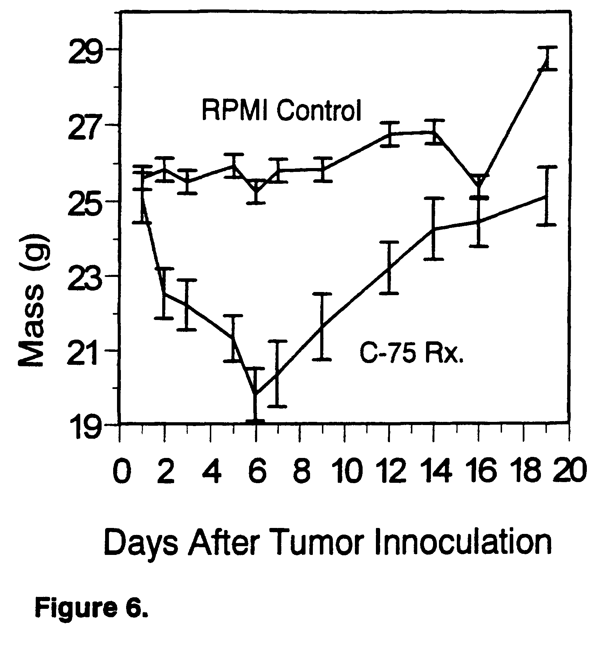

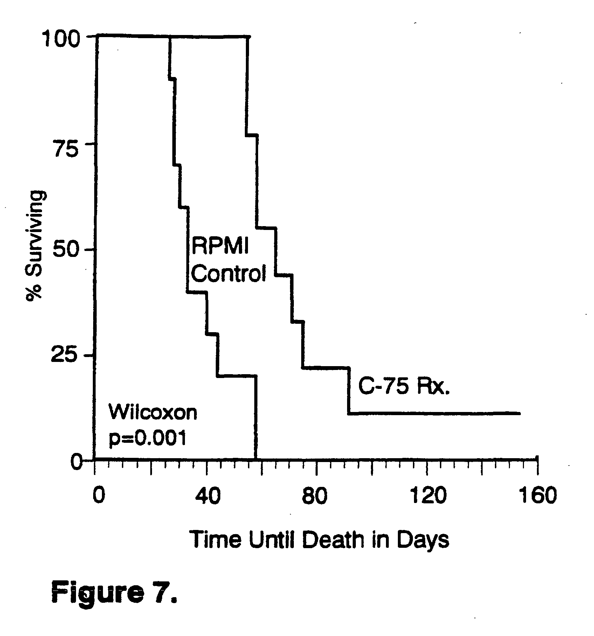

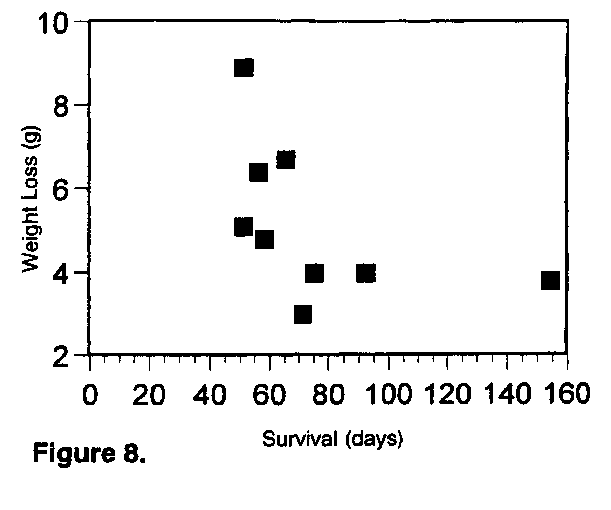

20 nude mice (Harland) mice were inoculated on Day 0 with 0.1 ml packed OVCAR-3 cells. 10 mice received transisomer of C-75 (20 mg/kg) in 100 ul RPMI on days 1, 3, and 5. 10 mice received 100 ul of RPMI i.p. alone on days 1, 3, and 5. Animals were weighed on days indicated on Figure 6. Animals were sacrificed when 30% increase in body weight occurred from tumor growth. C-75 causes weight loss in mice as shown in Figure 6. As shown in the Survival Curve in Figure 7, C-75 causes increased survival of the xenograft (p=0.001 Wilcoxon and Mantel Life Table Statistics.) Figure 8 is a plot of maximum weight loss vs. survival for individual mice, and this plot demonstrates that there is no correlation between survival and amount of weight loss for individual mice.

### Example 9. Dose response for C-75.

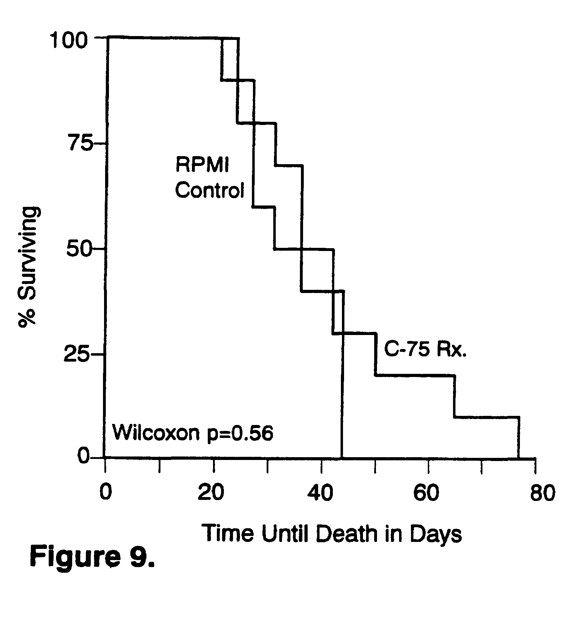

Figure 9 shows the results for an experiment performed as described for Example 8, except only one dose of C-75 was given. Survival is plotted on Figure 9, and there is a trend showing increased survival, but it was not statistically significant (wilcoxon p=0.56, Mantel p=0.52). This indicates a dose response to the anti-tumor effect of C-75. These mice showed an average maximum weight loss of about 3 grams on day 3.

## Claims

1. A pharmaceutical composition comprising a 5-substituted-2-oxo-3-methylene-4-furancarboxylic acid, wherein the substituent is selected from:
(a) a saturated linear alkyl group of 3-18 carbons;
(b) a saturated branched alkyl group of 3-18 carbons;
(c) an unsaturated linear or branched alkyl group of 3-18 carbons;
(d) 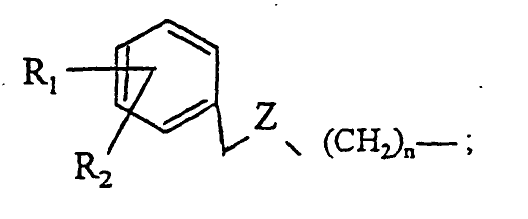
(e) 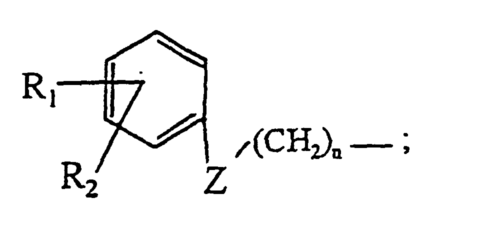
(f) 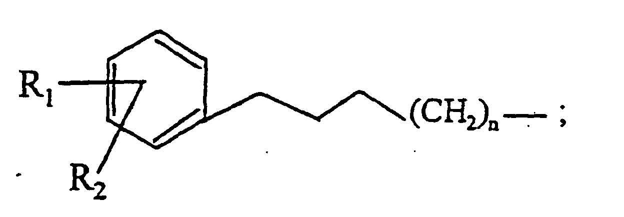
(g) 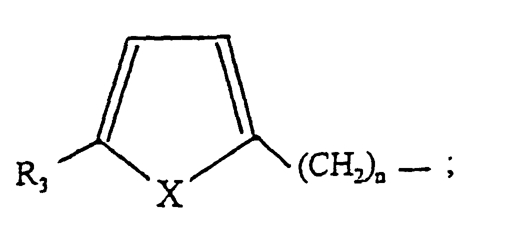
(h) 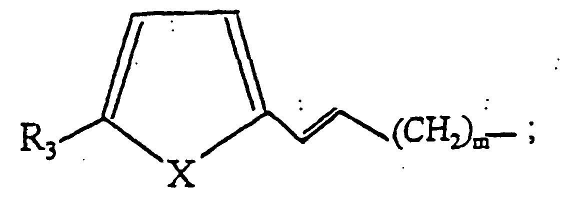
(i) 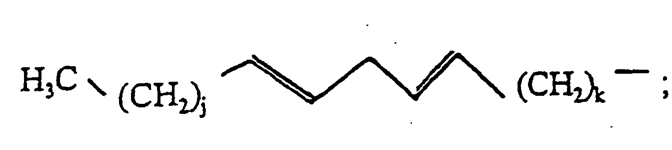 and
(j) 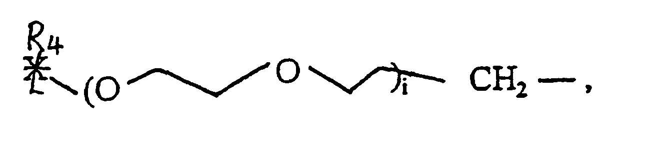
wherein R₁ and R₂ each are H, CH₃, C₂H₅, C₃H₇, C₄H₉, CF₃, OCH₃, F, Cl, or Br; R₃ is H, CH₃, C₂H₅, C₃H₇, C₄H₉, COOH, COOCH₃, COOC₂H₅, COOC₃H₇, or COOC₄H₉; R₄ is H, CH₃, C₂H₅, C₃H₇, C₄H₉; X is NH, S or O; Z is CH₂, O, NH or S; i is 1 to 5; j is 0 to 10; k is 1 to 10; m is 1-13; and n is 1 to 15, and R₁ and R₂ may be the same or different, wherein the 5-substituted-2-oxo-3-methylene-4-furancarboxylic acid is not methylenolactin.

2. The composition of claim 1 in which the substituent is C₃H₇, C₄H₉, C₆H₁₃, C₇H₁₅, C₈H₁₇, C₉H₁₉, C₁₀H₂₁, C₁₂H₂₅, C₁₄H₂₉, C₁₅H₃₁, C₁₆H₃₃, C₁₇H₃₅, or C₁₈H₃₇.

3. The composition of claim 1 in which the substituent is n-octyl.

4. The composition of claim 1 in which the substituent is 3E-4-(3-fluorophenyl) or 3E,6E-octadienyl.

5. A non-therapeutic method for inducing weight loss in an animal comprising administering to the animal a pharmaceutical composition comprising a 5-substituted-2-oxo-3-methylene-4-furancarboxylic acid, wherein the substituent is selected from:
(a) a saturated linear alkyl group of 3-18 carbons;
(b) a saturated branched alkyl group of 3-18 carbons;
(c) an unsaturated linear or branched alkyl group of 3-18 carbons;
(d) 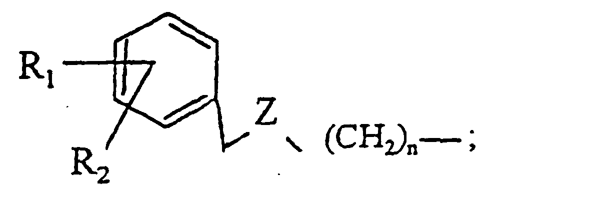
(e) 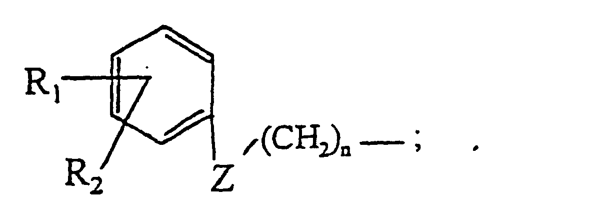
(f) 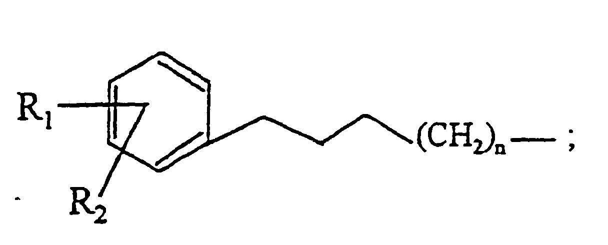
(g) 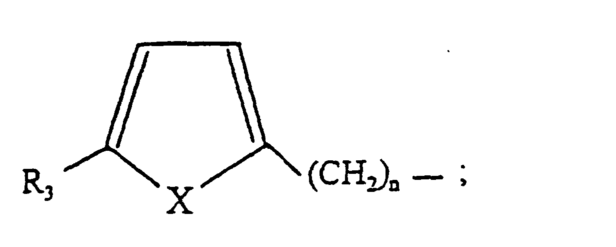
(h) 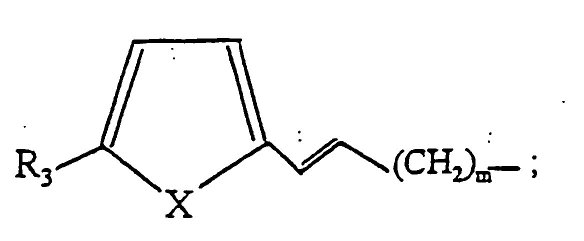
(i) 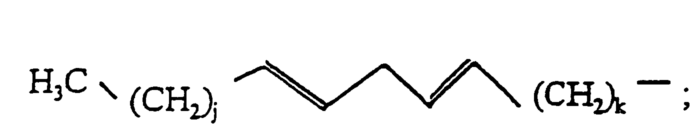 and
(j) 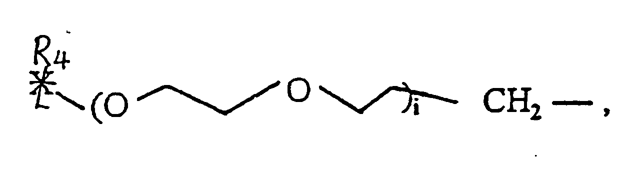
wherein R₁ and R₂ each are H, CH₃, C₂H₅, C₃H₇, C₄H₉, CF₃, OCH₃, F, C1, or Br; R₃ is H, CH₃, C₂H₅, C₃H₇, C₄H₉, COOH, COOCH₃, COOC₂H₅, COOC₃H₇, or COOC₄H₉; R₄ is H, CH₃, C₂H₅, C₃H₇, C₄H₉; X is NH, S or O; Z' is CH₂, O, NH or S; i is '1 to 5; j is 0 to 10; k is 1 to 10; m is 1-13; and n is 1 to 15, and R₁ and R₂ may be the same or different.

6. The composition according to claims 1 to 4 for use in therapy.

7. The use of a pharmaceutical composition comprising a 5-substituted-2-oxo-3-methylene-4-furancarboxylic acid, wherein the substituent is selected from:
(a) a saturated linear alkyl group of 3-18 carbons;
(b) a saturated branched alkyl group of 3-18 carbons;
(c) an unsaturated linear or branched alkyl group of 3-18 carbons;
(d) 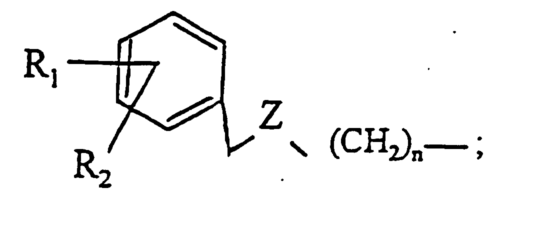
(e) 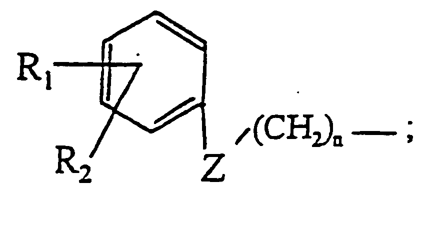
(f) 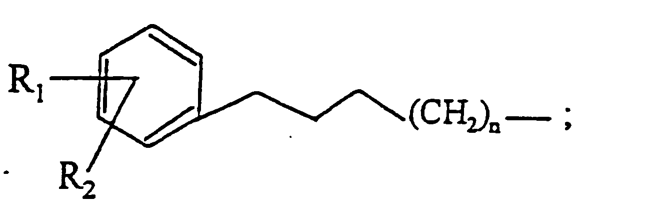
(g) 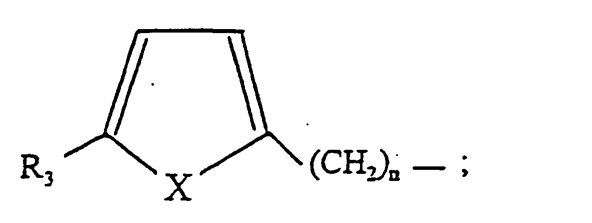
(h) 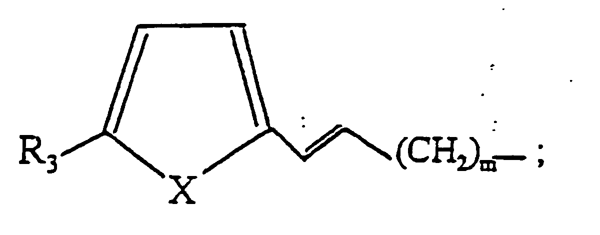
(i) 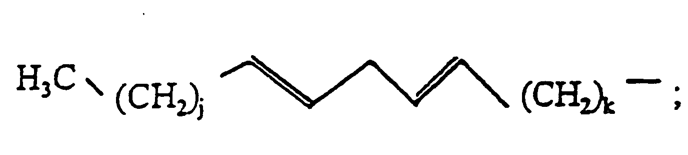 and
(j) 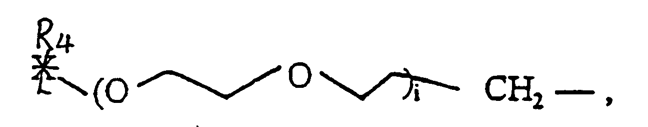
wherein R₁ and R₂ each are H, CH₃, C₂H₅, C₃H₇, C₄H₉, CF₃, OCH₃, F, C1, or Br; R₃ is H, CH₃, C₂H₅, C₃H₇, C₄H₉, COOH, COOCH₃, COOC₂H₅, COOC₃H₇, or COOC₄H₉; R₄ is H, CH₃, C₂H₅, C₃H₇, C₄H₉; X is NH, S or O; Z' is CH₂, O, NH or S; i is 1 to 5; j is 0 to 10; k is 1 to 10; m is 1-13; and n is 1 to 15, and R₁ and R₂ may be the same or different.
in the preparation of a medicament for the treatment of a condition responsive to reduction in adipose tissue mass in an animal or for treatment to induce weight loss in an animal.

8. Use of a pharmaceutical composition comprising a 5-substituted-2-oxo-3-methylene-4-furancarboxylic acid, wherein the substituent is selected from:
(a) a saturated linear alkyl group of 3-18 carbons;
(b) a saturated branched alkyl group of 3-18 carbons;
(c) an unsaturated linear or branched alkyl group of 3-18 carbons;
(d) 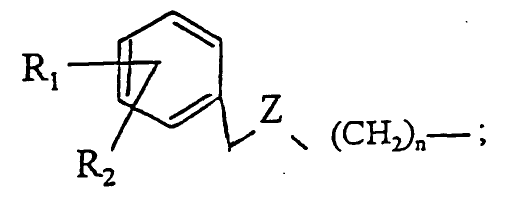
(e) 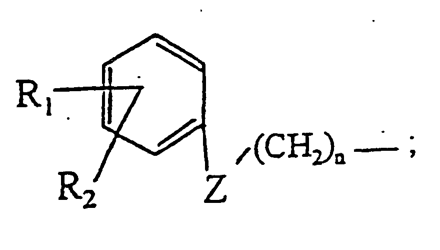
(f) 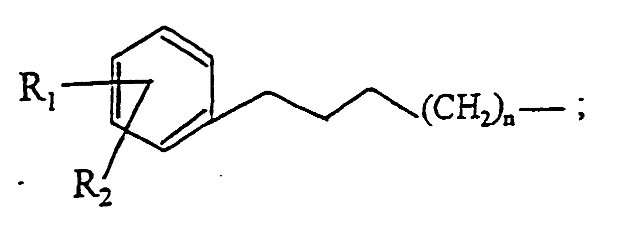
(g) 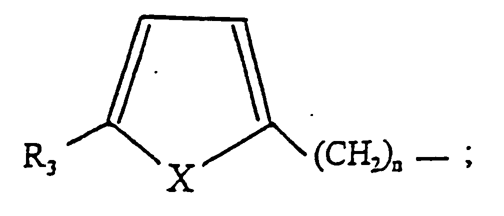
(h) 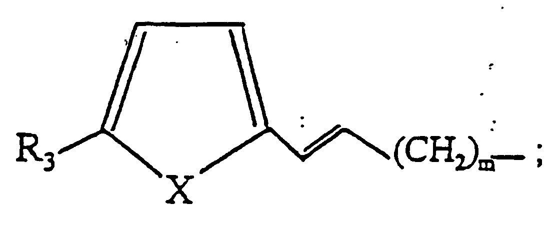
(i) 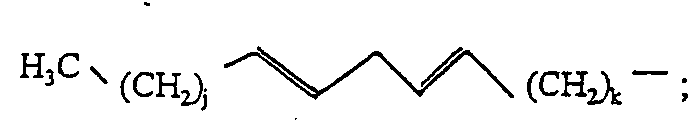 and
(j) 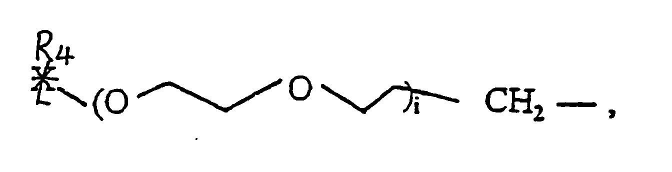
wherein R₁ and R₂ each are H, CH₃, C₂H₅, C₃H₇, C₄H₉, CF₃, OCH₃, F, Cl, or Br; R₃ is H, CH₃, C₂H₅, C₃H₇, C₄H₉, COOH, COOCH₃, COOC₂H₅, COOC₃H₇, or COOC₄H₉; R₄ is H, CH₃, C₂H₅, C₃H₇, C₄H₉; X is NH, S or O; Z is CH₂, O, NH or S; i is 1 to 5; j is 0 to 10; k is 1 to 10; m is 1-13; and n is 1 to 15, and R₁ and R₂ may be the same or different,
in the preparation of a medicament for the treatment of tumour cells expressing at least one enzyme of the fatty acid biosynthetic pathway.

9. The use of a pharmaceutical composition comprising a 5-substituted-2-oxo-3-methylene-4-furancarboxylic acid, wherein the substituent is selected from:
(a) a saturated linear alkyl group of 3-18 carbons;
(b) a saturated branched alkyl group of 3-18 carbons;
(c) an unsaturated linear or branched alkyl group of 3-18 carbons;
(d) 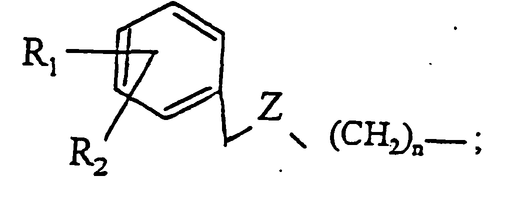
(e) 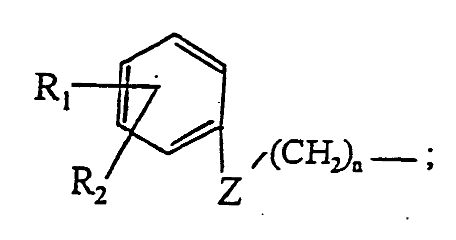
(f) 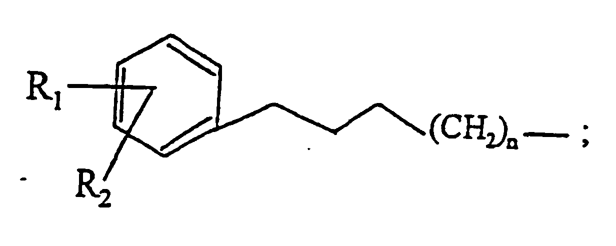
(g) 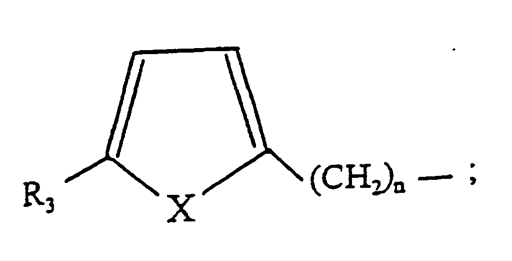
(h) 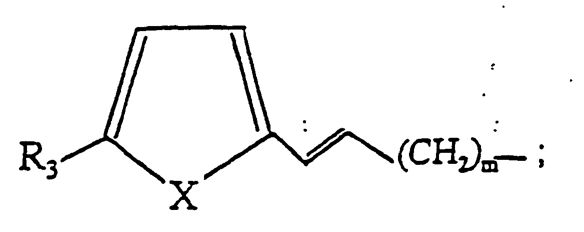
(i) 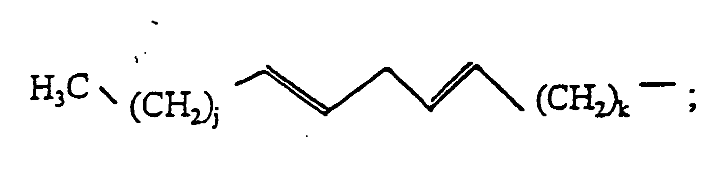 and
(j) 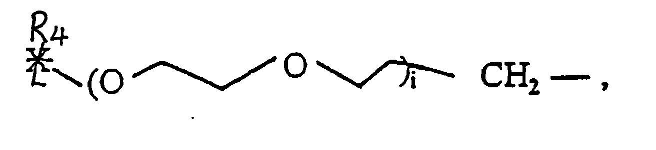
wherein R₁ and R₂ each are H, CH₃, C₂H₅, C₃H₇, C₄H₉, CF₃, OCH₃, F, Cl, or Br; R₃ is H, CH₃, C₂H₅, C₃H₇, C₄H₉, COOH, COOCH₃, COOC₂H₅, COOC₃H₇, or COOC₄H₉; R₄ is H, CH₃, C₂H₅, C₃H₇, C₄H₉; X is NH, S or O; Z' is CH₂, O, NH or S; i is 1 to 5; j is 0 to 10; k is 1 to 10; m is 1-13; and n is 1 to 15, and R₁ and R₂ may be the same or different,
in the preparation of a medicament for the treatment of tumour cells expressing fatty acid synthase (FAS).

## Patentansprüche

1. Pharmazeutische Zusammensetzung, umfassend eine 5-substituierte 2-Oxo-3-methylen-4-furancarbonsäure, wobei der Substituent ausgewählt ist aus:
(a) einer gesättigten geradkettigen Alkylgruppe mit 3-18 Kohlenstoffen;
(b) einer gesättigten verzweigten Alkylgruppe mit 3-18 Kohlenstoffen;
(c) einer ungesättigten geradkettigen oder verzweigten Alkylgruppe mit 3-18 Kohlenstoffen;
(d) 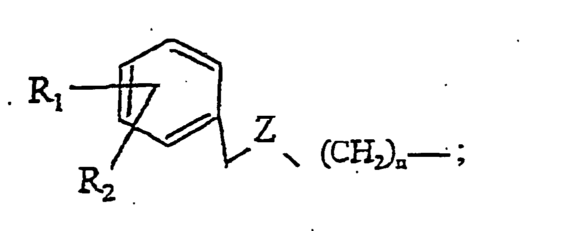
(e) 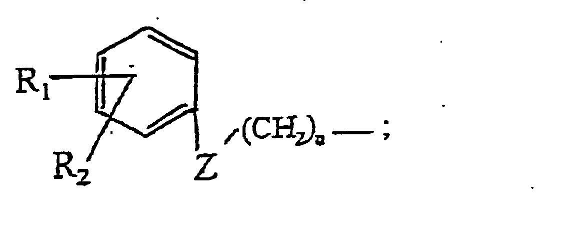
(f) 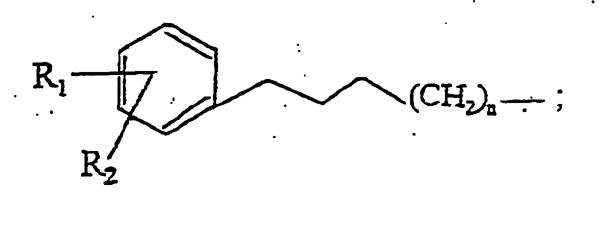
(g) 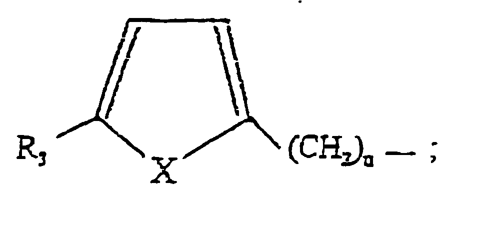
(h) 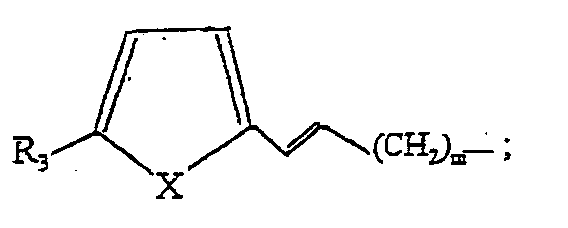
(i) 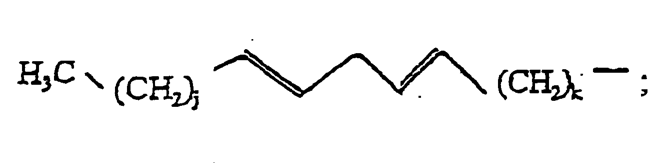 und
(j) 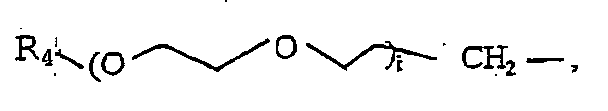
wobei R₁ und R₂ jeweils H, CH₃, C₂H₅, C₃H₇, C₄H₉, CF₃, OCH₃, F, Cl oder Br sind; R₃ H, CH₃, C₂H₅, C₃H₇, C₄H₉, COOH, COOCH₃, COOC₂H₅, COOC₃H₇ oder COOC₄H₉ ist; R₄ H, CH₃, C₂H₅, C₃H₇, C₄H₉ ist; X NH, S oder O ist; Z CH₂, O, NH oder S ist; i 1 bis 5 ist; j 0 bis 10 ist; k 1 bis 10 ist; m 1 bis 13 ist; und n 1 bis 15 ist, und R₁ und R₂ gleich oder verschieden sein können, wobei die 5-substituierte 2-Oxo-3-mathylen-4-furancarbonsäure nicht Methylenolactin ist.

2. Zusammensetzung nach Anspruch 1, wobei der Substituent C₃H₇, C₄H₉, C₆H₁₃, C₇H₁₅, C₈H₁₇, C₉H₁₉, C₁₀H₂₁, C₁₂H₂₅, C₁₄H₂₉, C₁₅H₃₁, C₁₆H₃₃, C₁₇H₃₅ oder C₁₈H₃₇ ist.

3. Zusammensetzung nach Anspruch 1, wobei der Substituent n-Octyl ist.

4. Zusammensetzung nach Anspruch 1, wobei der Substituent 3E-4-(3-fluorphenyl) oder 3E,6E-Octadienyl ist.

5. Nicht-therapeutisches Verfahren zum Induzieren von Gewichtsverlust bei einem Lebewesen, umfassend das Verabreichen einer pharmazeutischen Zusammensetzung an das Lebewesen, die eine 5-substituierte 2-Oxo-3-methylen-4-furancarbonsäure umfasst, wobei der Substituent ausgewählt ist aus:
(a) einer gesättigten geradkettigen Alkylgruppe mit 3-18 Kohlenstoffen;
(b) einer gesättigten verzweigten Alkylgruppe mit 3-18 Kohlenstoffen;
(c) einer ungesättigten geradkettigen oder verzweigten Alkylgruppe mit 3-18 Kohlenstoffen;
(d) 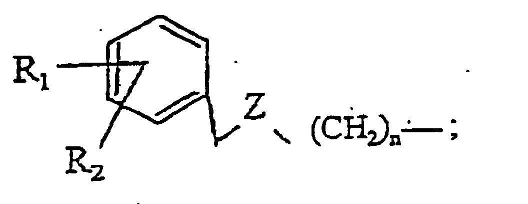
(e) 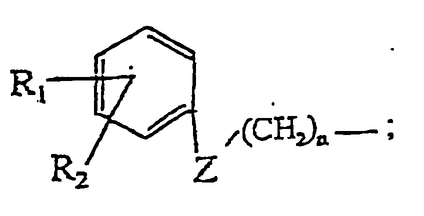
(f) 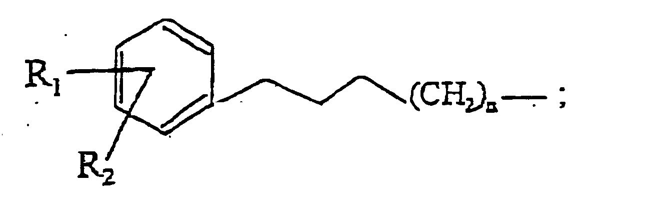
(g) 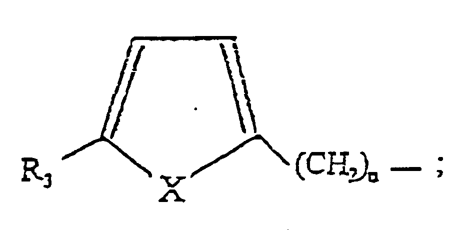
(h) 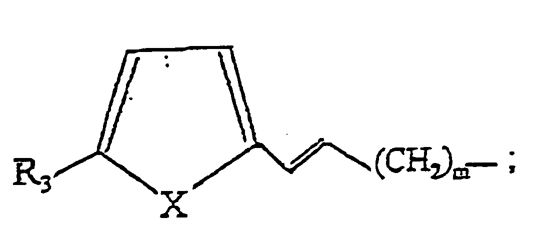
(i) 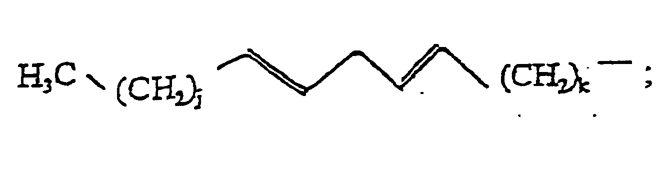 und
(j) 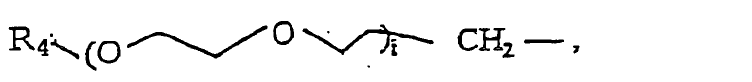
wobei R₁ und R₂ jeweils H, CH₃, C₂H₅, C₃H₇, C₄H₉, CF₃, OCH₃, F, Cl oder Br sind; R₃ H, CH₃, C₂H₅, C₃H₇, C₄H₉, COOH, COOCH₃, COOC₂H₅, COOC₃H₇ oder COOC₄H₉ ist; R₄ H, CH₃, C₂H₅, C₃H₇, C₄H₉ ist; X NH, S oder 0 ist; Z CH₂, O, NH oder S ist; i 1 bis 5 ist; j 0 bis 10 ist; k 1 bis 10 ist; m 1 bis 13 ist; und n 1 bis 15 ist, und R₁ und R₂ gleich oder verschieden sein können.

6. Zusammensetzung nach Anspruch 1 bis 4 zur therapeutischen Verwendung.

7. Verwendung einer pharmazeutischen Zusammensetzung, die eine 5-substituierte 2-Oxo-3-methylen-4-furancarbonsäure umfasst, wobei der Substituent ausgewählt ist aus:
(a) einer gesättigten geradkettigen Alkylgruppe mit 3-18 Kohlenstoffen;
(b) einer gesättigten verzweigten Alkylgruppe mit 3-18 Kohlenstoffen;
(c) einer ungesättigten geradkettigen oder verzweigten Alkylgruppe mit 3-18 Kohlenstoffen;
(d) 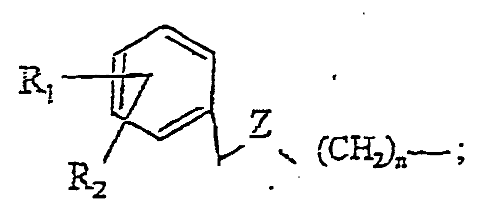
(e) 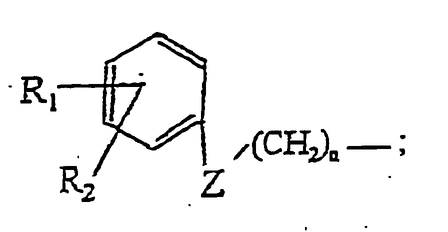
(f) 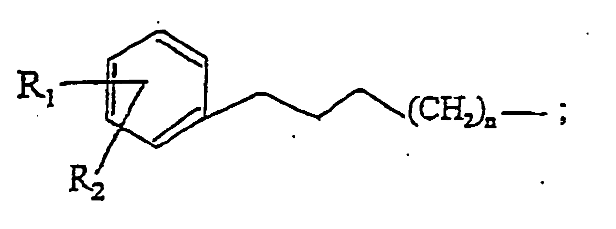
(g) 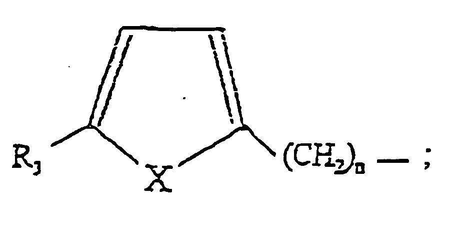
(h) 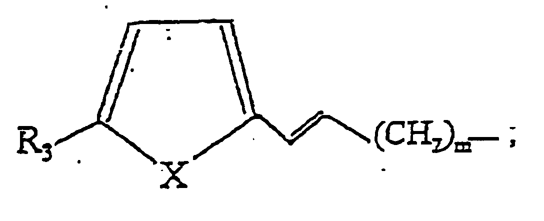
(i) 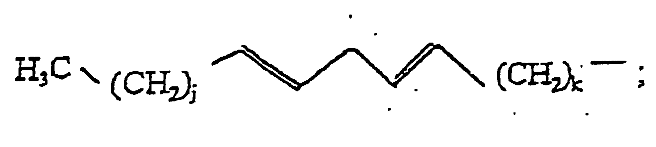 und
(j) 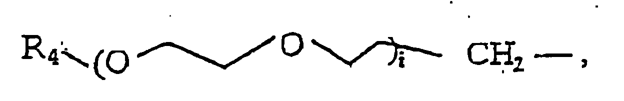
wobei R₁ und R₂ jeweils H, CH₃, C₂H₅, C₃H₇, C₄H₉, CF₃, OCH₃, F, Cl oder Br sind; R₃ H, CH₃, C₂H₅, C₃H₇, C₄H₉, COOH, COOCH₃, COOC₂H₅, COOC₃H₇ oder COOC₄H₉ ist; R₄ H, CH₃, C₂H₅, C₃H₇, C₄H₉ ist; X NH, S oder O ist; Z CH₂, O, NH oder S ist; i 1 bis 5 ist; j 0 bis 10 ist; k 1 bis 10 ist; m 1 bis 13 ist; und n 1 bis 15 ist, und R₁ und R₂ gleich oder verschieden sein können,
zur Herstellung eines Medikaments zur Behandlung eines auf Abnahme von Fettgewebemasse reagierenden Zustands bei einem Lebewesen oder zur Behandlung zum Induzieren von.Gewichtsverlust bei einem Lebewesen.

8. Verwendung einer pharmazeutischen Zusammensetzung, die eine 5-substituierte 2-Oxo-3-methylen-4-furancarbonsäure umfasst, wobei der Substituent ausgewählt ist aus:
(a) einer gesättigten geradkettigen Alkylgruppe mit 3-18 Kohlenstoffen;
(b) einer gesättigten verzweigten Alkylgruppe mit 3-18 Kohlenstoffen;
(c) einer ungesättigten geradkettigen oder verzweigten Alkylgruppe mit 3-18 Kohlenstoffen;
(d) 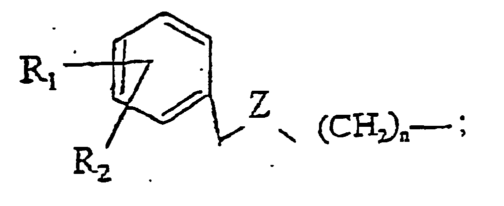
(e) 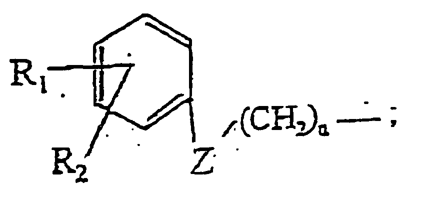
(f) 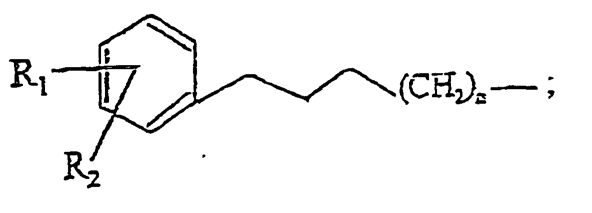
(g) 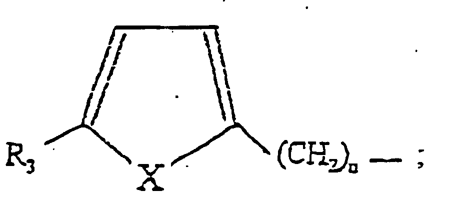
(h) 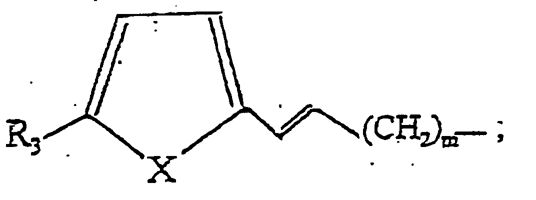
(i) 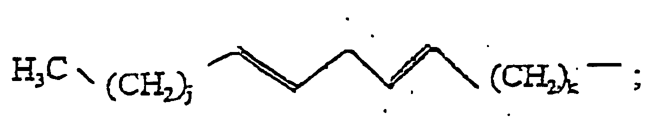 und
(j) 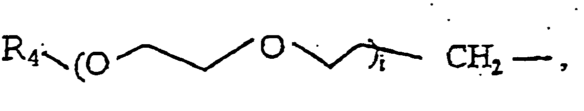
wobei R₁ und R₂ jeweils H, CH₃, C₂H₅, C₃H₇, C₄H₉, CF₃, OCH₃, F, Cl oder Br sind; R₃ H, CH₃, C₂H₅, C₃H₇, C₄H₉, COOH, COOCH₃, COOC₂H₅, COOC₃H₇ oder COOC₄H₉ ist; R₄ H, CH₃, C₂H₅, C₃H₇, C₄H₉ ist; X NH, S oder O ist; Z CH₂, O, NH oder S ist; i 1 bis 5 ist; j 0 bis 10 ist; k 1 bis 10 ist; m 1 bis 13 ist; und n 1 bis 15 ist, und R₁ und R₂ gleich oder verschieden sein können,
zur Herstellung eines Medikaments zur Behandlung von Tumorzellen, die wenigstens ein Enzym des Fettsäurebiosynthesewegs exprimieren.

9. Verwendung einer pharmazeutischen Zusammensetzung, die eine 5-substituierte 2-Oxo-3-methylen-4-furancarbonsäure umfasst, wobei der Substituent ausgewählt ist aus:
(a) einer gesättigten geradkettigen Alkylgruppe mit 3-18 Kohlenstoffen;
(b) einer gesättigten verzweigten Alkylgruppe mit 3-18 Kohlenstoffen;
(c) einer ungesättigten geradkettigen oder verzweigten Alkylgruppe mit 3-18 Kohlenstoffen;
(d) 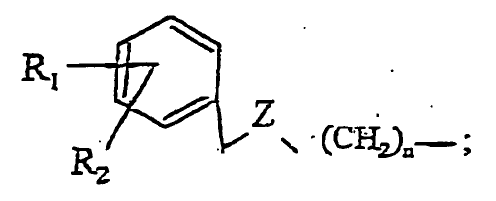
(e) 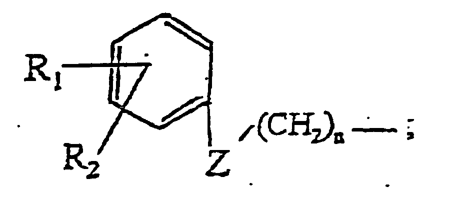
(f) 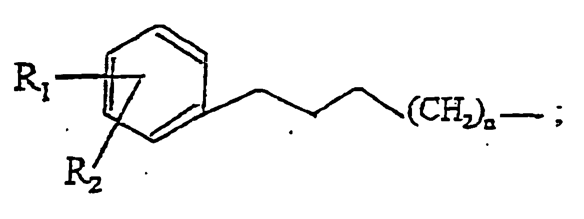
(g) 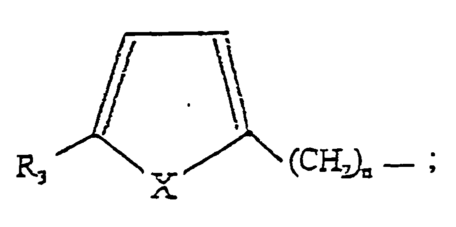
(h) 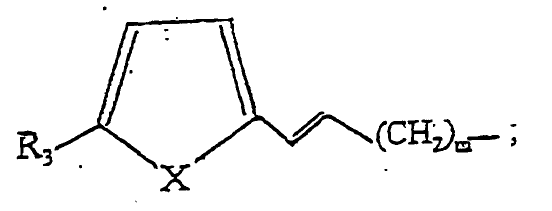
(i) 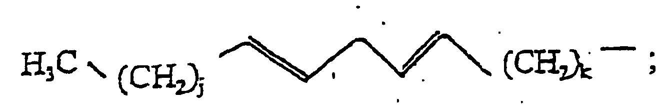 und
(j) 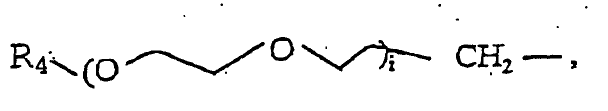
wobei R₁ und R₂ jeweils H, CH₃, C₂H₅, C₃H₇, C₄H₉, CF₃, OCH₃, F, Cl oder Br sind; R₃ H, CH₃, C₂H₅, C₃H₇, C₄H₉, COOH, COOCH₃, COOC₂H₅, COOC₃H₇ oder COOC₄H₉ ist; R₄ H, CH₃, C₂H₅, C₃H₇, C₄H₉ ist; X NH, S oder O ist; Z CH₂, O, NH oder S ist; i 1 bis 5 ist; j 0 bis 10 ist; k 1 bis 10 ist; m 1 bis 13 ist; und n 1 bis 15 ist, und R₁ und R₂ gleich oder verschieden sein können,
zur Herstellung eines Medikaments zur Behandlung von Tumorzellen, die Fettsäuresynthase (FAS) exprimieren.

## Revendications

1. Composition pharmaceutique comprenant un acide 5-substitué-2-oxo-3-méthylène-4-furanecarboxylique, où le substituant est choisi parmi :
(a) un groupe alkyle linéaire saturé de 3-18 carbones ;
(b) un groupe alkyle ramifié saturé de 3-18 carbones ;
(c) un groupe alkyle linéaire ou ramifié insaturé de 3-18 carbones ;
(d) 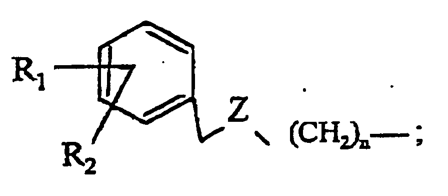
(e) 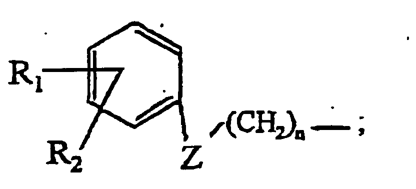
(f) 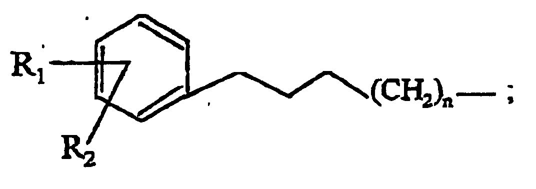
(g) 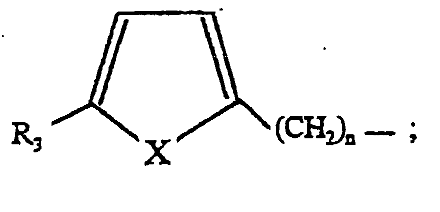
(h) 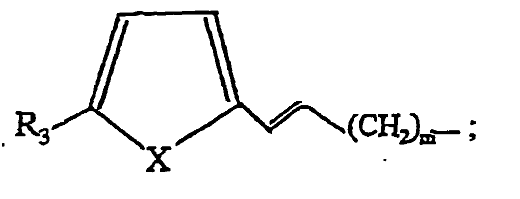
(i) 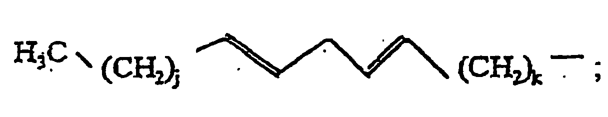 et
(j) 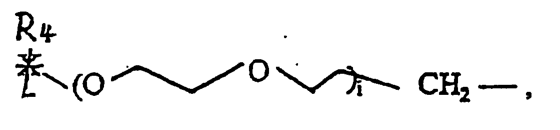
où R₁ et R₂ sont chacun H, CH₃, C₂H₅, C₃H₇, C₄H₉, CF₃, OCH₃, F, Cl ou Br ; R₃ est H, CH₃, C₂H₅, C₃H₇, C₄H₉, COOH, COOCH₃, COOC₂H₅, COOC₃H₇, ou COOC₄H₉ ; R₄ est H, CH₃, C₂H₅, C₃H₇, C₄H₉ ; X est NH, S ou O ; Z est CH₂, O, NH ou S ; i est 1 à 5 ; j est 0 à 10 ; k est 1 à 10 ; m est 1-13 ; et n est 1 à 15, et R₁ et R₂ peuvent être identiques ou différents, où l'acide 5-substitué-2-oxo-3-méthylène-4-furanecarboxylique n'est pas la méthylénolactine.

2. Composition selon la revendication 1 où le substituant est C₃H₇, C₄H₉, C₆H₁₃, C₇H₁₅, C₈H₁₇, C₉H₁₉, C₁₀H₂₁, C₁₂H₂₅, C₁₄H₂₉, C₁₅H₃₁, C₁₆H₃₃, C₁₇H₃₅, ou C₁₈H₃₇.

3. Composition selon la revendication 1 où le substituant est n-octyle.

4. Composition selon la revendication 1 où le substituant est 3E-4-(3-fluorophényle) ou 3E,6E-octadiényle.

5. Procédé non thérapeutique pour induire une perte de poids chez un animal comprenant l'administration à l'animal d'une composition pharmaceutique comprenant un acide 5-substitué-2-oxo-3-méthylène-4-furanecarboxylique, où le substituant est choisi parmi :
(a) un groupe alkyle linéaire saturé de 3-18 carbones ;
(b) un groupe alkyle ramifié saturé de 3-18 carbones ;
(c) un groupe alkyle linéaire ou ramifié insaturé de 3-18 carbones ;
(d) 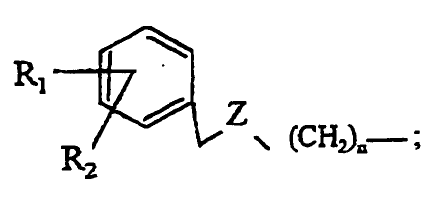
(e) 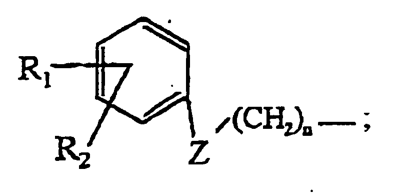
(f) 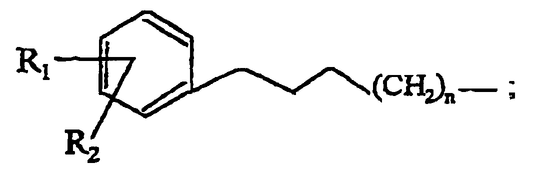
(g) 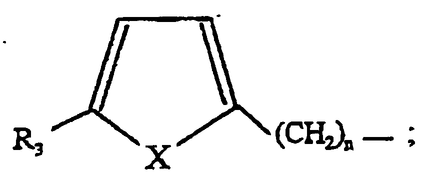
(h) 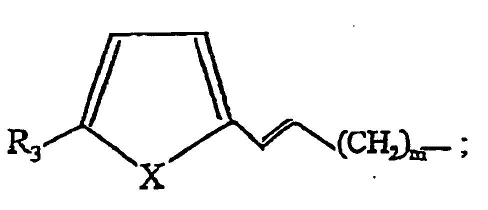
(i) 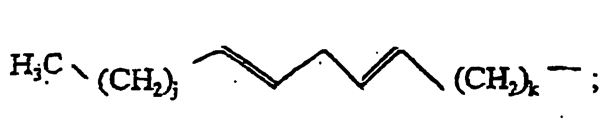 et
(j) 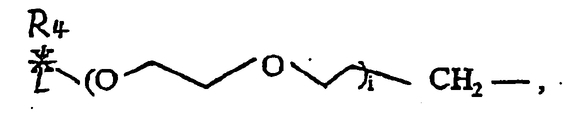
où R₁ et R₂ sont chacun H, CH₃, C₂H₅, C₃H₇, C₄H₉, CF₃, OCH₃, F, Cl ou Br; R₃ est H, CH₃, C₂H₅, C₃H₇, C₄H₉, COOH, COOCH₃, COOC₂H₅, COOC₃H₇, ou COOC₄H₉ ; R₄ est H, CH₃, C₂H₅, C₃H₇, C₄H₉ ; X est NH, S ou O ; Z est CH₂, O, NH ou S ; i est 1 à 5 ; j est 0 à 10 ; k est 1 à 10 ; m est 1-13 ; et n est 1 à 15, et R₁ et R₂ peuvent être identiques ou différents.

6. Composition selon les revendications 1 à 4 destinée à être utilisée en thérapie.

7. Utilisation d'une composition pharmaceutique comprenant un acide 5-substitué-2-oxo-3-méthylène-4-furanecarboxylique, où le substituant est choisi parmi :
(a) un groupe alkyle linéaire saturé de 3-18 carbones ;
(b) un groupe alkyle ramifié saturé de 3-18 carbones ;
(c) un groupe alkyle linéaire ou ramifié insaturé de 3-18 carbones ;
(d) 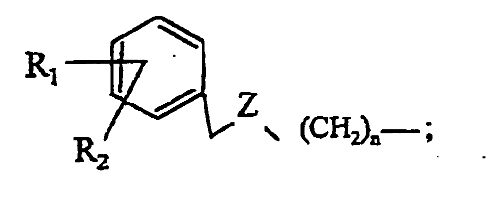
(e) 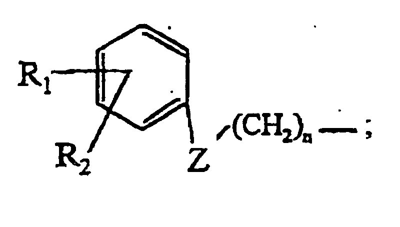
(f) 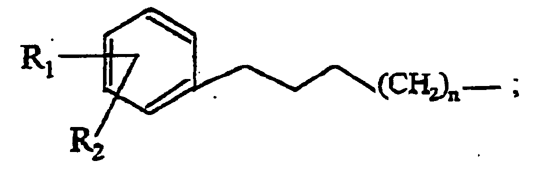
(g) 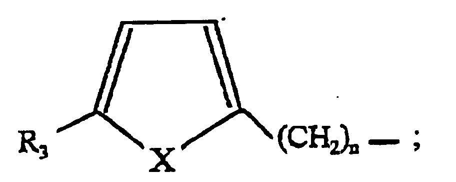
(h) 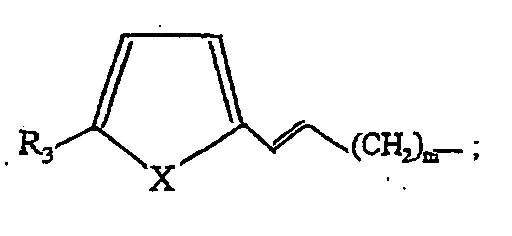
(i) 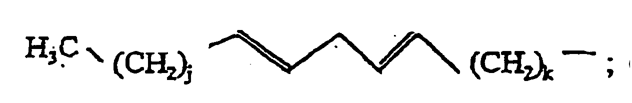 et
(j) 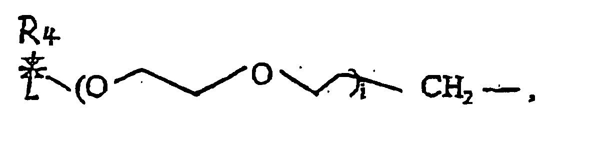
où R₁ et R₂ sont chacun H, CH₃, C₂H₅, C₃H₇, C₄H₉, CF₃, OCH₃, F, Cl ou Br ; R₃ est H, CH₃, C₂H₅, C₃H₇, C₄H₉, COOH, COOCH₃, COOC₂H₅, COOC₃H₇, ou COOC₄H₉; R₄ est H, CH₃, C₂H₅, C₃H₇, C₄H₉; X est NH, S ou O ; Z est CH₂, O, NH ou S ; i est 1 à 5; j est 0 à 10; k est 1 à 10; m est 1-13 ; et n est 1 à 15, et R₁ et R₂ peuvent être identiques ou différents,
dans la préparation d'un médicament pour le traitement d'une affection sensible à une réduction de la masse de tissu adipeux chez un animal ou pour un traitement pour induire une perte de poids chez un animal.

8. Utilisation d'une composition pharmaceutique comprenant un acide 5-substitué-2-oxo-3-méthylène-4-furanecarboxylique, où le substituant est choisi parmi :
(a) un groupe alkyle linéaire saturé de 3-18 carbones ;
(b) un groupe alkyle ramifié saturé de 3-18 carbones ;
(c) un groupe alkyle linéaire ou ramifié insaturé de 3-18 carbones ;
(d) 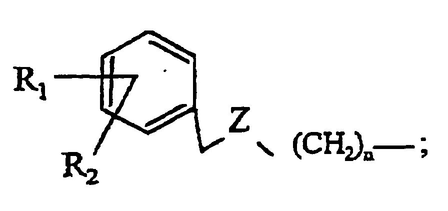
(e) 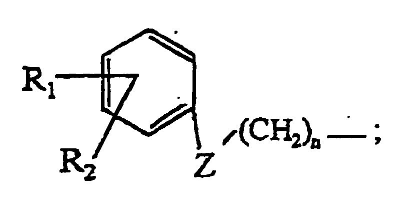
(f) 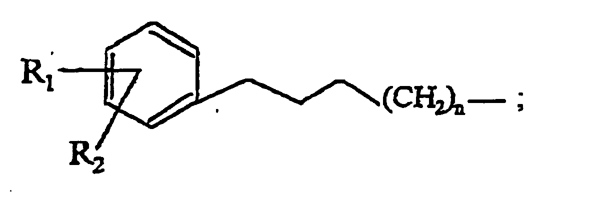
(g) 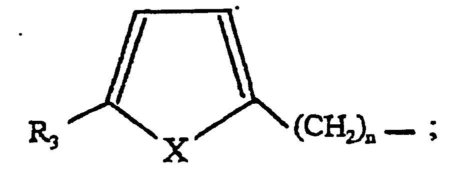
(h) 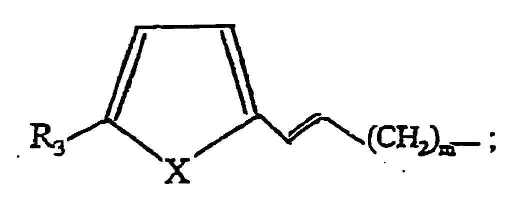
(i) 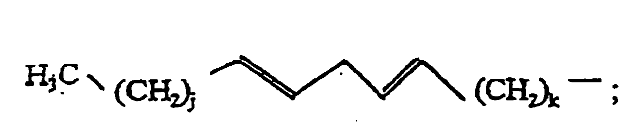 et
(j) 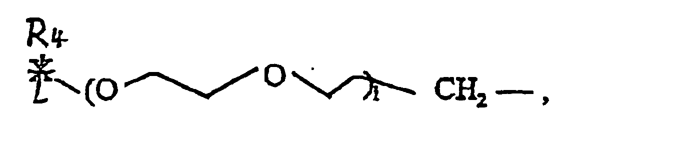
où R₁ et R₂ sont chacun H, CH₃, C₂H₅, C₃H₇, C₄H₉, CF₃, OCH₃, F, Cl ou Br ; R₃ est H, CH₃, C₂H₅, C₃H₇, C₄H₉, COOH, COOCH₃, COOC₂H₅, COOC₃H₇, ou COOC₄H₉; R₄ est H, CH₃, C₂H₃, C₃H₇, C₄H₉ ; X est NH, S ou O ; Z est CH₂, O, NH ou S; i est 1 à 5 ; j est 0 à 10 ; k est 1 à 10 ; m est 1-13 ; et n est 1 à 15, et R₁ et R₂ peuvent être identiques ou différents,
dans la préparation d'un médicament pour le traitement de cellules tumorales exprimant au moins une enzyme de la voie de biosynthèse des acides gras.

9. Utilisation d'une composition pharmaceutique comprenant un acide 5-substitué-2-oxo-3-méthylène-4-furanecarboxylique, où le substituant est choisi parmi :
(a) un groupe alkyle linéaire saturé de 3-18 carbones ;
(b) un groupe alkyle ramifié saturé de 3-18 carbones ;
(c) un groupe alkyle linéaire ou ramifié insaturé de 3-18 carbones ;
(d) 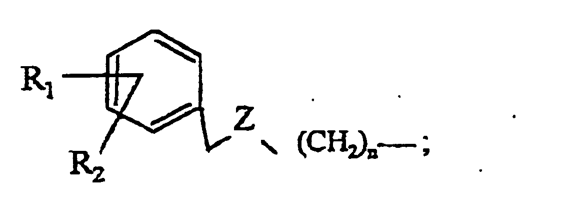
(e) 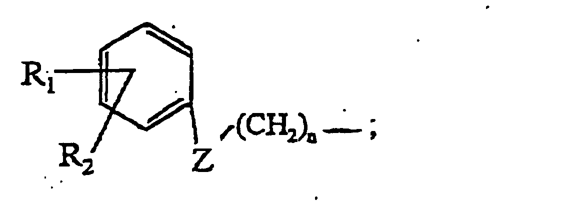
(f) 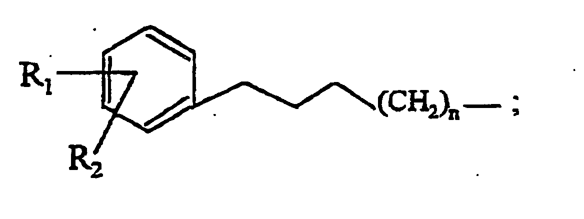
(g) 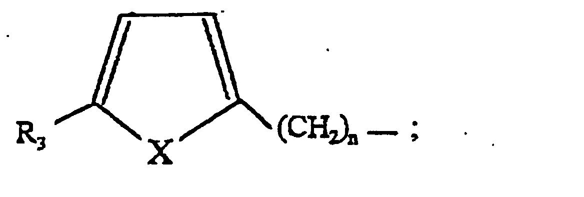
(h) 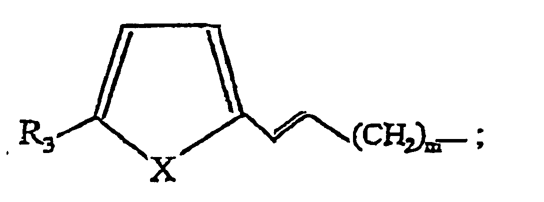
(i) 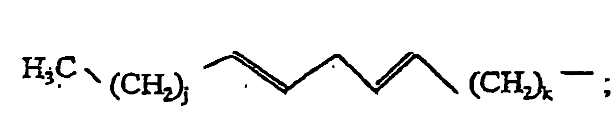 et
(j) 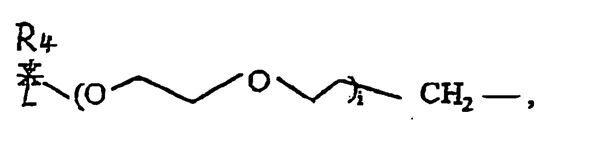
où R₁ et R₂ sont chacun H, CH₃, C₂H₅, C₃H₇, C₄H₉, CF₃, OCH₃, F, Cl ou Br ; R₃ est H, CH₃, C₂H₅, C₃H₇, C₄H₉, COOH, COOCH₃, COOC₂H₅, COOC₃H₇, ou COOC₄H₉; R₄ est H, CH₃, C₂H₅, C₃H₇, C₄H₉; X est NH, S ou O ; Z est CH₂, O, NH ou S ; i est 1 à 5 ; j est 0 à 10 ; k est 1 à 10 ; m est 1-13 ; et n est 1 à 15, et R₁ et R₂ peuvent être identiques ou différents,
dans la préparation d'un médicament pour le traitement de cellules tumorales exprimant l'acide gras synthase (FAS).
